# FASCICULE DE BREVET EUROPEEN

(11) **EP 0 632 017 B1**
(45) Date de publication et mention de la délivrance du brevet: **06.05.1998**
(21) Numéro de dépôt: 94401458.8
(22) Date de dépôt: 28.06.1994
(51) Int. Cl.: C07C 255/20, C07C 255/21, C07C 255/23, C07C 255/31, C07C 255/30, C07C 255/28, A61K 31/275

(54) **Nouveaux N-phényl 2-cyano 3-hydroxy propénamides, leurs formes tautomères et leurs sels, leur procédé de préparation, l'application à titre de médicaments de ces nouveaux produits et les compositions les renfermant**
N-Phenyl-2-Cyano-3-hydroxypropenamide, ihre Tautomeren, ihre Salze, Verfahren zu ihrer Herstellung, ihre Anwendung als Medikamente und Zusammensetzungen, die sie enthalten
N-phenyl-2-cyano-3-hydroxy propenamides, their tautomeres and salts, their use as medicaments and compositions containing them

(30) Priorité: 29.06.1993 GB 9313365
(43) Date de publication de la demande: 04.01.1995
(73) Titulaire: HOECHST MARION ROUSSEL, 92800 Puteaux (FR)
(72) Inventeur: Kuo, Elizabeth Anne, Swindon,Wiltshire SN3 5PH (GB)
(74) Mandataire: Vieillefosse, Jean-Claude

(56) Documents cités:
- EP-A- 0 551 230
- WO-A-91/17748
- DE-B- 2 524 929
- JOURNAL OF THE CHEMICAL SOCIETY. PERKIN TRANACTIONS 1, vol.1992, no.17, LETCHWORTH GB pages 2203 - 2213 C.A. AXTON ET AL

## Description

La présente invention concerne de nouveaux N-phenyl 2-cyano-3-hydroxy-propenamides, leurs formes tautomères et leurs sels, ainsi que le procédé de préparation, l'application à titre de médicaments de ces nouveaux produits et les compositions les renfermant.

La demande de brevet internationale WO-A-91 17748 décrit des dérivés du 2-cyano-3-hydroxypropénamide. Par ailleurs des N-phényl-2-cyano-3-hydroxypropénamides sont décrits dans la demande de brevet allemand DE-B-2524929.

L'invention a pour objet de nouveaux N-phenyl 2-cyano-3-hydroxy-propenamides répondant à la formule générale (I) : 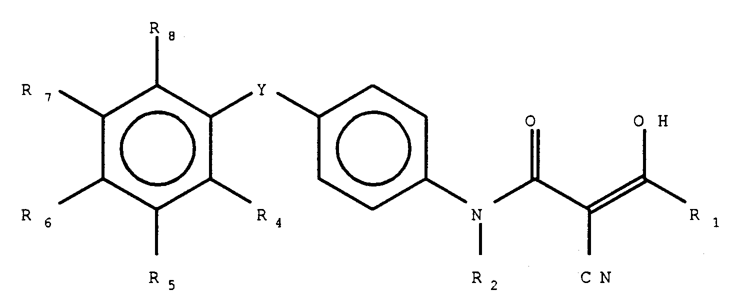 dans laquelle :
- R₁ représente un radical cycloalkyle renfermant de 3 à 6 atomes de carbone,
- R₂ représente un atome d'hydrogène, un radical alkyle renfermant de 1 à 3 atomes de carbone,
- Y représente un groupement:
- NR₃- , -SO₂-NR₃- -NR₃-SO₂- , -CHOH- , -CF₂- , -(CH₂)ₘ- , 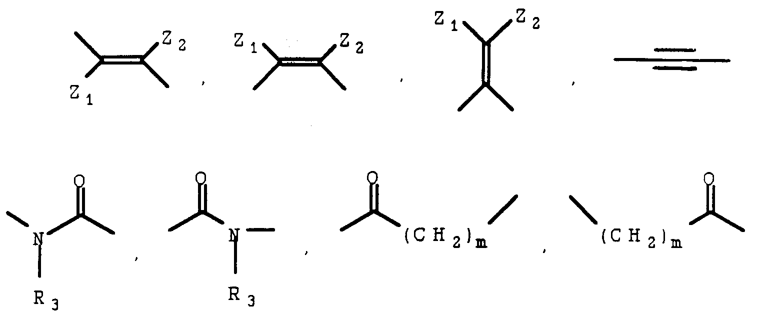 groupements dans lesquels Z₁ et Z₂, identiques ou différents, représentent un atome d'hydrogène, un atome d'halogène, un radical alkyle renfermant de 1 à 3 atomes de carbone, un groupement -CN , un groupement - NO₂ , R₃ représente un atome d'hydrogène ou un radical alkyle renfermant de 1 à 3 atomes de carbone, m est un nombre entier qui peut varier de 0 à 6 ,
- R₄, R₅, R₆, R₇ et R₈, identiques ou différents, représentent un atome d'hydrogène, un atome d'halogène, un groupement -CN, un groupement -NO₂, un radical alkyle, linéaire ou ramifié, renfermant de 1 à 6 atomes de carbone, un radical cycloalkyle renfermant de 3 à 6 atomes de carbone, un radical alcoxy, linéaire ou ramifié, renfermant de 1 à 6 atomes de carbone, un radical alkylthio renfermant de 1 à 6 atomes de carbone, un radical -(CH₂)ₙ-CX₃ , un radical -O-(CH₂)ₙ-CX₃ , un radical -S-(CH₂)ₙ-CX₃ , radicaux dans lesquels n représente un nombre entier compris entre 0 et 3 et X représente un atome d'halogène, ou un groupement - CO - R₁₀ dans lequel R₁₀ représente un atome d'hydrogène, un radical alkyle, linéaire ou ramifié, renfermant de 1 à 6 atomes de carbone, un radical cycloalkyle renfermant de 3 à 6 atomes de carbone, ou R₆ et R₇ , forment ensemble un groupement -O-CH₂-O- , et R₄, R₅ et R₈ , ont la définition indiquée ci-dessus, leurs formes tautomères, ainsi que leurs sels d'addition avec les bases minérales ou organiques.

Dans la formule générale (I) et dans ce qui suit :
- par radical alkyle renfermant de 1 à 3 atomes de carbone, on entend un radical méthyle, éthyle, propyle, isopropyle;
- par radical alkyle renfermant de 1 à 6 atomes de carbone, on entend de préférence un radical méthyle, éthyle, propyle, isopropyle, butyle linéaire ou ramifié, pentyle linéaire ou ramifié, hexyle linéaire ou ramifié,
- par radical cycloalkyle renfermant de 3 à 6 atomes de carbone, on entend un radical cyclopropyle, cyclobutyle, cyclopentyle ou cyclohexyle;
- par atome d'halogène, on entend un atome de fluor, de chlore, de brome ou d'iode et de préférence un atome de fluor, de chlore ou de brome;
- par radical alcoxy comportant de 1 à 6 atomes de carbone, on entend par exemple, un radical méthoxy, éthoxy, propoxy, isopropoxy, butoxy linéaire ou ramifié, pentyloxy linéaire ou ramifié, hexyloxy linéaire ou ramifié;
- par radical alkylthio comportant de 1 à 6 atomes de carbone, on entend par exemple, un radical méthylthio, éthylthio, propylthio, isopropylthio, butylthio linéaire ou ramifié, pentylthio linéaire ou ramifié, hexylthio linéaire ou ramifié,
- par radicaux -(CH₂)ₙ-CX₃ , -O-(CH₂)ₙ-CX₃ , -S-(CH₂)ₙ-CX₃ dans lesquels n représente un nombre entier compris entre 0 et 3, on entend par exemple des radicaux :
   -CF₃ , -(CH₂)-CF₃ , -(CH₂)₂-CF₃ , -(CH₂)₃-CF₃ , -O-CF₃ , -O-(CH₂)-CF₃ , -O-(CH₂)₂-CF₃ , -O-(CH₂)₃-CF₃ , -S-CF₃ , -S-(CH₂)-CF₃ , -S-(CH₂)₂-CF₃ , -S-(CH₂)₃-CF₃.

Le groupement de formule : 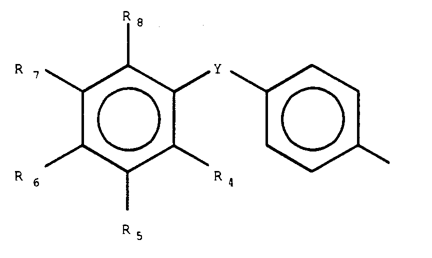 peut notamment représenter un groupement 4-(4'-chlorobenzoyl)-phenyl-, 4-[N-(4-chlorophenyl)-N-methyl-aminocarbonyl]-phenyl-, 4-(4'-chlorophenyl-E-ethenyl)-phenyl, 4-(4'-chlorophenyl-ethynyl)-phenyl- , 4-(4'-chlorophenyl-Z-ethenyl)-phenyl-, 4-(4'-fluorophenyl-E-ethenyl)-phenyl-, 4-(4'-fluorophenyl-ethynyl)-phenyl]-, 4-(phenylethyl)-phenyl-, 4-biphenylyl- and 4-(4'-fluorophenyl)-phenyl-.

Les sels d'addition avec les bases minérales ou organiques peuvent être, par exemple, les sels formés avec les bases minérales tels que les sels de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris(hydroxyméthyl) aminométhane, l'éthanolamine, la pyridine, la picoline, la dicyclohexylamine, la morpholine, la benzylamine, la procaïne, la lysine, l'arginine, l'histidine, la N-méthylglucamine.

Parmi les produits, objet de l'invention, on peut citer notamment les dérivés répondant à la formule (I) ci-dessus, ainsi que leurs sels, caractérisés en ce que dans ladite formule (I), R₁ représente un radical cyclopropyle, Y, R₂, R₄, R₅, R₆, R₇ et R₈ ont la signification déjà indiquée, ainsi que leurs sels d'addition avec les bases minérales ou organiques.

Parmi ces derniers, on peut citer plus particulièrement les dérivés répondant à la formule (I) ci-dessus, caractérisés en ce que dans ladite formule (I),
R₂ représente un atome d'hydrogène ou un radical méthyle, et R₁, Y, R₄, R₅, R₆, R₇ et R₈ ont la signification déjà indiquée, ainsi que leurs sels d'addition avec les bases minérales ou organiques.

Parmi ces derniers on retient plus particulièrement les dérivés de formule (I) dans laquelle :
R₁ représente un radical cyclopropyle,
R₂ représente un atome d'hydrogène ou un radical méthyle,
Y représente un groupement -(CH₂)ₘ- dans lequel m est égal à 0, 1 ou 2 ou un groupement: 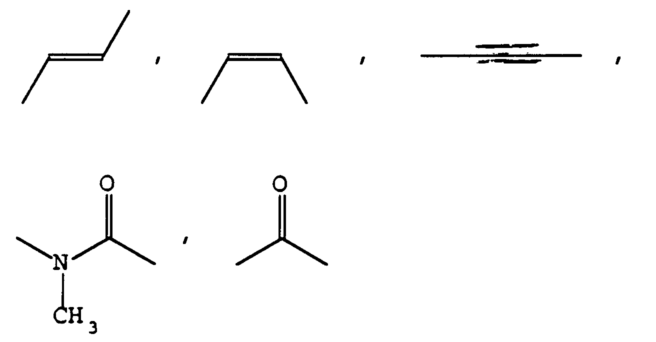 et soit R₄, R₅, R₆, R₇ et R₈ , identiques ou différents, représentent un atome d'hydrogène, un atome de chlore, de brome ou de fluor, un groupement -CN , un groupement -NO₂ , un radical méthyle, un radical cyclopropyle, un radical méthoxy, un radical méthylthio, un radical -(CH₂)ₙ-CF₃ , un radical -O-(CH₂)ₙ-CF₃ , un radical -S-(CH₂)ₙ-CF₃ , radicaux dans lesquels n représente un nombre entier compris entre 0 et 3, ou un groupement -CO-R₁₀ dans lequel R₁₀ représente un atome d'hydrogène, un radical méthyle, un radical cyclopropyle, soit R₆ et R₇ , forment ensemble un groupement 6,7-O-CH₂-O-, et R₄, R₅ et R₈ ont la signification déjà indiquée, leurs formes tautomères, ainsi que leurs sels d'addition avec les bases minérales ou organiques et,
   tout particulèrement, les dérivés de formule (I) dans laquelle
R₁ représente un radical cyclopropyle, R₂ représente un atome d'hydrogène ou un radical méthyle,
Y représente un groupement -(CH₂)ₘ- dans lequel m est égal à 0, 1 ou 2 ou un groupement : 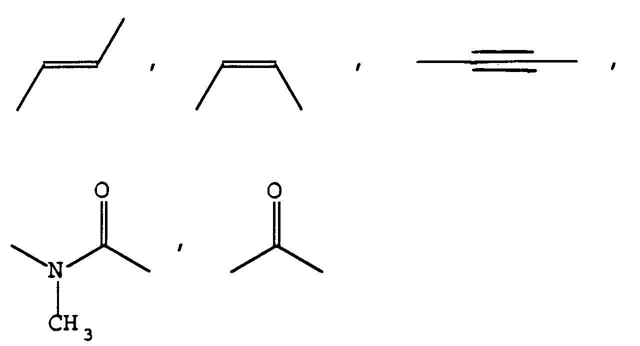
R₄, R₅, R₆, R₇ et R₈ , identiques ou différents, représentent un atome d'hydrogène, un atome de chlore, de brome ou de fluor, un groupement - NO₂ , un radical méthyle ou un radical -CF₃ , leurs formes tautomères, ainsi que leurs sels d'addition avec les bases minérales ou organiques.

Parmi ces derniers, on retient tout particulièrement les dérivés de formule (I) dont les noms suivent :
- N-[4-(4'-chlorophenyl-E-ethenyl) phenyl] 2-cyano 3-cyclopropyl 3-hydroxy-prop-2-enamide,
- N-[4-(4'-fluorophenyl-E-ethenyl) phenyl] 2-cyano 3-cyclopropyl 3-hydroxy-prop-2-enamide,
- N-[4-biphenylyl] 2-cyano 3-cyclopropyl 3-hydroxy-prop-2-enamide,
- N-[4-(4'-fluorophenyl) phenyl] 2-cyano 3-cyclopropyl 3-hydroxy-prop-2-enamide, ainsi que leurs sels d'addition avec les bases minérales ou organiques.

L'invention a également pour objet un procédé de préparation des nouveaux N-phenyl 2-cyano-3-hydroxy-propenamides tels que définis par la formule (I) ci-dessus, ainsi que de leurs sels, caractérisé en ce que l'on fait réagir un produit de formule (II) : 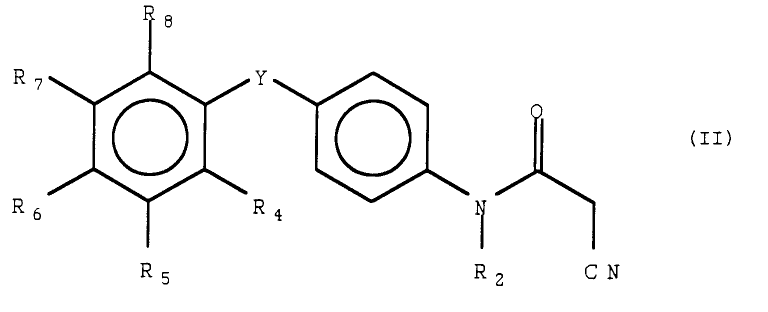 dans laquelle R₂, Y, R₄, R₅, R₆, R₇ et R₈, ont la signification déjà indiquée, avec successivement de l'hydrure de sodium, si nécessaire en présence d'un catalyseur, puis fait réagir le produit avec le dérivé fonctionnel d'un acide de formule (III): 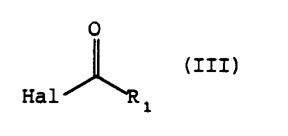 dans laquelle Hal représente un atome d'halogène et R₁ a la signification déjà indiquée, pour obtenir un produit de formule (I) correspondant que l'on isole et si désiré salifie ou bien avec le dérivé fonctionnel d'un acide de formule (III_{A}) : 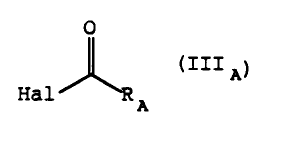 dans laquelle Hal représente un atome d'halogène et R_{A} représente un radical R₁ dûment protégé, pour obtenir un produit de formule (I_{A}): 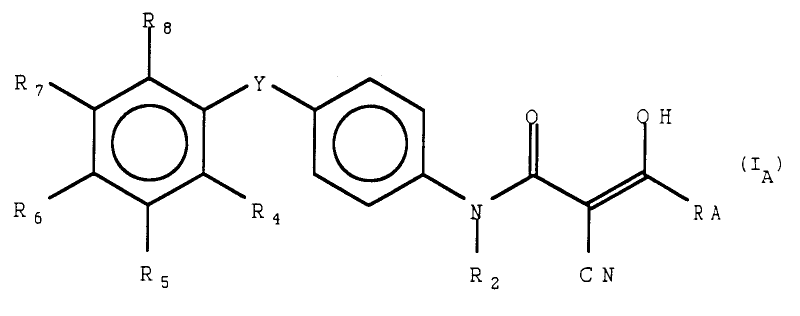 dans laquelle R_{A}, R₂, Y, R₄, R₅, R₆, R₇ et R₈ ont la signification déjà indiquée, puis, déprotège le produit de formule (I_{A}) ainsi obtenu dans lequel R_{A} a la signification déjà indiquée, pour obtenir un produit de formule (I) correspondant, que l'on isole et si désiré salifie.

Dans des conditions préférentielles de mise en oeuvre de l'invention, le procédé de préparation ci-dessus décrit est caractérisé en ce que :
- la réaction du produit de formule (II) avec l'hydrure de sodium est effectuée au sein d'un solvant organique anhydre tel que le tétrahydrofuranne ou le dichloromethane en présence si nécessaire d'un catalyseur capable de solvater l'hydrure de sodium comme par exemple l'imidazole,
- la réaction avec le dérivé fonctionnel de l'acide de formule (III) ou (III_{A}) est effectuée de préférence avec le chlorure ou le fluorure d'acide et de préférence en présence d'imidazole, au sein d'un solvant organique anhydre tel que le tétrahydrofuranne ou le dichlorométhane,
- la réaction avec le dérivé fonctionnel de l'acide de formule (III) ou (III_{A}) est effectuée à basse température ou à température ambiante,
- le dérivé fonctionnel de l'acide de formule (III) ou (III_{A}) peut être par exemple le fluorure de propynoyl, préparé in situ par action d'acide propiolique sur du fluorure de benzoyle,
- le radical R₁ peut être protégé par un groupement arylseleno, de préférence un groupement phenylseleno,
- la déprotection peut être effectuée par oxydation au moyen d'un peroxyde tel que le peroxyde d'hydrogène en l'absence de solvants organiques ou au sein d'un solvant ou d'un mélange de solvants organiques tel que le mélange methanol-dichloromethane.

Les produits de formule (I) présentent un caractère acide. On peut avantageusement préparer les sels d'addition des produits de formule (I) en faisant réagir, en proportions sensiblement stoechiométriques, une base minérale ou organique avec lesdits produits de formule (I). Les sels peuvent être préparés sans isoler les acides correspondants.

Les produits, objet de la présente invention, possèdent de très intéressantes propriétés pharmacologiques. On note en particulier une remarquable activité anti-inflammatoire. Ils inhibent d'une part les phénomènes inflammatoires provoqués par des agents irritants, et d'autre part les réactions d'hypersensibilité retardée, en empéchant l'activation des cellules immunitaires par un antigène spécifique.

Ces propriétés sont illustrées plus loin dans la partie expérimentale.

Ces propriétés justifient l'utilisation des nouveaux N-phenyl 2-cyano-3-hydroxy-propenamides répondant à la formule (I), ainsi que de leurs sels d'addition avec les bases pharmaceutiquement acceptables, à titre de médicaments.

La présente invention a aussi également pour objet l'application à titre de médicaments des nouveaux N-phenyl 2-cyano-3-hydroxy-propenamides tels que définis par la formule générale (I), ainsi que de leurs sels d'addition avec les bases minérales ou organiques pharmaceutiquement acceptables.

Parmi les médicaments, objet de l'invention, on retient notamment les médicaments, caractérisés en ce qu'ils sont constitués par les nouveaux N-phenyl 2-cyano-3-hydroxy-propenamides répondant à la formule (I) dans laquelle R₁ représente un radical cyclopropyle ou des radicaux de formule : 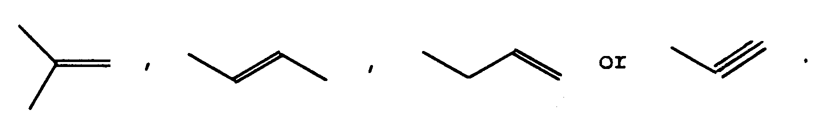 Y, R₂, R₄, R₅, R₆, R₇ et R₈ ont la signification déjà indiquée,
ainsi que leurs sels d'addition avec les bases minérales ou organiques pharmaceutiquement acceptables.
Parmi les médicaments, objet de l'invention, on retient plus particulièrement ceux répondant à la formule (I) ci-dessus dans laquelle R₂ représente un atome d'hydrogène ou un radical méthyle,
Y, R₁, R₄, R₅, R₆, R₇ et R₈ ont la signification déjà indiquée,
ainsi que leurs sels d'addition avec les bases minérales ou organiques pharmaceutiquement acceptables.

Parmi les médicaments préférés de l'invention, on retient tout particulièrement les produits de formule (I) dans laquelle R₁ représente un radical cyclopropyle, R₂ représente un atome d'hydrogène ou un radical méthyle, Y represente un groupement -(CH₂)ₘ- dans lequel m est égal à 0, 1 ou 2 ou un groupement : 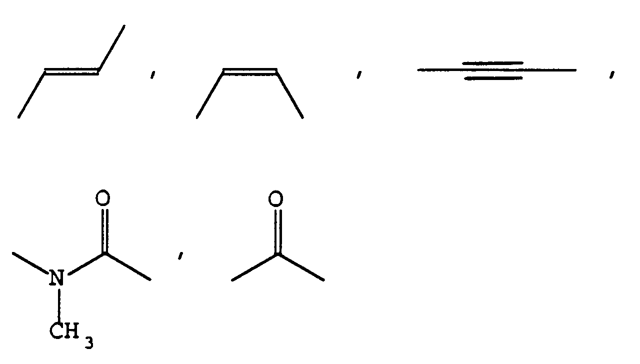 et soit R₄, R₅, R₆, R₇ et R₈ , identiques ou différents, représentent un atome d'hydrogène, un atome de chlore, de brome ou de fluor, un groupement -CN , un groupement -NO₂ , un radical méthyle, un radical cyclopropyle, un radical méthoxy, un radical méthylthio, un radical -(CH₂)ₙ-CF₃ , un radical -O-(CH₂)ₙ-CF₃ , un radical -S-(CH₂)ₙ-CF₃ , radicaux dans lesquels n représente un nombre entier compris entre 0 et 3, ou un groupement -CO-R₁₀ dans lequel R₁₀ représente un atome d'hydrogène, un radical méthyle, un radical cyclopropyle, soit R₆ et R₇ , forment ensemble un groupement 6,7-O-CH₂-O-, R₄, R₅ et R₈ ayant la signification déjà indiquée,
leurs formes tautomères, et notamment les dérivés de formule (I) dont les noms suivent:
- N-[4-(4'-chlorophenyl-E-ethenyl) phenyl] 2-cyano 3-cyclopropyl 3-hydroxy-prop-2-enamide,
- N-[4-(4'-fluorophenyl-E-ethenyl) phenyl] 2-cyano 3-cyclopropyl 3-hydroxy-prop-2-enamide,
- N-[4-(biphenylyl] 2-cyano 3-cyclopropyl 3-hydroxy-prop-2-enamide,
- N-[4-(4'-fluorophenyl) phenyl] 2-cyano 3-cyclopropyl 3-hydroxy-prop-2-enamide,
   ainsi que leurs sels d'addition avec les bases minérales ou organiques pharmaceutiquement acceptables.

Ces médicaments trouvent, par exemple, leur emploi dans le traitement de l'arthrite rhumatoïde et des maladies inflammatoires chroniques d'origine immune ou non (greffes, transplantations d'organes...).

La dose usuelle variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels d'addition avec les bases pharmaceutiquement acceptables à titre de principe actif.

A titre de médicaments, les dérivés répondant à la formule (I) et leurs sels d'addition avec les bases pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables ; elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (II) peuvent être préparés par réaction d'un produit de formule (IV) : 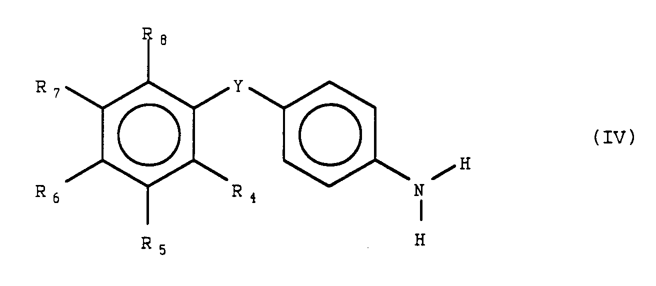 dans laquelle Y, R₄, R₅, R₆, R₇ et R₈ ont la signification déjà indiquée, avec de l'acide cyanoacétique et de préférence en présence de dicyclocarbodiimide ou de pentachlorure de phosphore et au sein d'un solvant organique anhydre tel que le tetrahydrofurane ou le dichloromethane.

Les produits de formule (IV), lorsqu'ils ne sont pas connus, peuvent être préparés par réduction du produit nitré correspondant de formule (V): 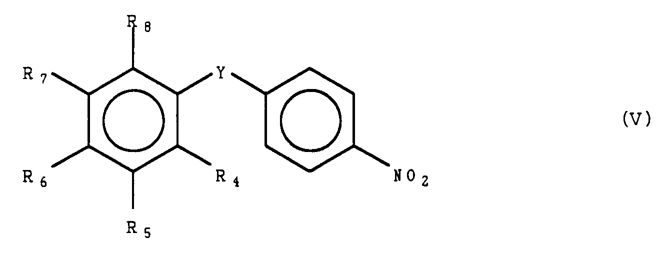 dans laquelle Y, R₄, R₅, R₆, R₇ et R₈ ont la signification déjà indiquée.

Des exemples de préparation de produits de formule (V) sont donnés plus loin dans la partie experimentale.

Les composés de formule (II) et (IV) tels que définis ci-dessus sont nouveaux et constituent d'autres aspects de la présente invention.

Ils sont utiles en tant que produits industriels et en particulier en tant qu'intermédiaires dans la préparation des composés de formule (I).

Il va être donné maintenant à titre non limitatif, des exemples de mise en oeuvre de l'invention.

### EXEMPLE 1: N-[4-(4'-Chlorobenzoyl)phényl]-2-cyano-3-cyclopropyl-3-hydroxy-prop-2-énamide

Du N-[4-(4'-chlorobenzoyl)phényl]cyanoacétamide (6,52 g, 21,8 mmoles) a été ajouté en plusieurs portions en 1 h à une suspension d'hydrure de sodium (80 % de dispersion dans l'huile, 1,97 g, 65,5 mmoles, 3 équiv) dans du tétrahydrofurane (100 ml). Le mélange a été agité pendant 1 h puis une solution de chlorure de cyclopropane carbonyle (3,42 g, 2,97 ml, 32,7 mmoles, 1,5 équiv) dans du THF (50 ml) a été ajoutée goutte à goutte. Le mélange a été agité pendant 30 min avant l'addition d'acide acétique (3,75 ml, 65,5 mmoles, 3 équiv) avec précaution pour arrêter la réaction. Après 20 min, le mélange réactionnel a été ajouté à un mélange, sous vive agitation, d'HCl 2M (100 ml) dans de l'eau glacée (400 ml). La suspension a été filtrée et le solide lavé à l'eau (2 × 50 ml) puis séché sous vide. Le solide a ensuite été agité dans de l'éther (150 ml) pendant 30 min et filtré. Le solide a été lavé à l'éther de pétrole 40-60 (2 × 30 ml) et séché sous vide pour donner 6,84 g (86 %) du produit recherché.
RMN ¹H δ (CDCl₃) 1,12-1,22 (2H, m), 1,28-1,39 (2H, m) 2,11-2,18 (1H, m), 7,03 (2H, s), 7,30-7,55 (9H, m), 15,85 (1H, s).

### Préparation de N-[4-(4'-chlorobenzoyl)phényl]cyanoacétamide.

### Etape 1: 4-chloro-4'-nitro-benzophénone

Du chlorure d'aluminium (14,7 g, 110 mmoles, 1,1 équiv) a été ajouté en portions en 40 min à une solution de chlorure de 4-nitro-benzoyl (18,6 g, 100 mmoles) dans du chlorobenzène (66 ml, 650 mmoles, 6,5 équiv). Le mélange a été agité à 50°C pendant 22 h, on l'a laissé refroidir jusqu'à la température ambiante et on l'a ajouté avec précaution à de l'acide chlorhydrique concentré (20 ml) dans de l'eau glacée (180 ml). Le mélange a été extrait avec de l'éther (4 × 100 ml). Les matières organiques réunies ont été lavées avec du KOH aqueux à 2 % (3 × 100 ml), de la saumure (75 ml), puis séchées (MgSO₄), filtrées et évaporées pour donner 22,2 g de poudre crème pâle. La chromatographie éclair sur colonne (2,5-15 % EtOAc/40-60 éther de pétrole) a donné 19,30 g (74 %) de produit a l'état de solide crème.
RMN ¹H (CDCl₃) δ 7,51 (2H, d), 7,76 (2H, d), 7,92 (2H, d) et 8,36 (2H, d).

### Etape 2: 4-amino-4'-chlorobenzophénone

Une suspension de 4-chloro-4'-nitrobenzophénone (14,4 g, 54,8 mmoles) et d'oxyde de platine (66,9 mg, 0,5 % molaire) a été agitée sous hydrogène dans de l'éthanol (300 ml) pendant 2 h. Le mélange a été filtré, évaporé et purifié par chromatographie éclair sur colonne (2-8 % EtOAc/CH₂Cl₂) pour donner 10,05 g (72 %) de solide jaune.
RMN ¹H (CDCl₃) δ 4,16 (2H, s large), 6,67 (2H, d), 7,43 (2H, d), 7,68 (2H, d), 7,69 (2H, d).

### Etape 3: N-[4-(4'-chlorobenzoyl)phényl]cyanoacétamide

De l'acide cyanoacétique (6,05 g, 71,1 mmoles, 1,5 équiv) a été ajouté en portions en 30 min à une suspension de pentachlorure de phosphore (14,8 g, 71,1 mmoles, 1,5 équiv) dans du dichlorométhane (150 ml). La solution a été portée au reflux pendant 30 min et une solution de 4-amino-4'-chlorobenzophénone (11,0 g, 47,4 mmoles) dans CH₂Cl₂ (50 ml) a ensuite été ajoutée. Le mélange a été porté au reflux pendant 2 h 20 min puis on l'a laissé refroidir jusqu'à la température ambiante. Une solution aqueuse saturée en bicarbonate de sodium (200 ml) a ensuite été ajoutée avec précaution. Après agitation pendant 1 h, le mélange a été filtré et le solide lavé à l'eau (2 × 50 ml) et séché sous vide; 13,14 g (93 %) de produit.
RMN ¹H (CDCl₃/CD₃OD) δ 3,64 (2H, s), 7,48 (2H, d), 7,70 (2H, d), 7,73 (2H, d), 7,80 (2H, d).

### EXEMPLE 2: N-[4-(N'-(4-chlorophényl)-N'-méthylaminocarbonyl)-phényl]-2-cyano-3-cyclopropyl-3-hydroxy-prop-2-énamide

En opérant selon un procédé analogue à celui mis en oeuvre dans l'exemple 1, on a obtenu le composé cité ci-dessus dans un rendement de 86 %.
RMN ¹H (CDCl₃) δ 1,10-1,23 (2h, m), 1,27-1,36 (2h, m), 2,06-2,18 (1H, m), 3,47 (3H, s), 6,97 (2H, d), 7,21 (2H, d), 7,30 (2H, d), 7,37 (2H, d), 7,58 (1H, s), 15,66 (1H, s).

### Préparation de N-[4-(N'-(4-chlorophényl)-N'-méthylaminocarbonyl)phényl]cyanoacétamide

### Etape 1: N-(4-chlorophényl)-N-méthyl-4-nitrobenzamide.

Une solution de N-méthyl-4-chloroaniline (6,53 g, 46,1 mmoles) dans du chloroforme (50 ml) a été ajoutée goutte à goutte en 80 min à du chlorure de 4-nitrobenzoyle (8,55 g, 46,1 mmoles) dans du chloroforme (150 ml). Une solution aqueuse saturée en bicarbonate de sodium (100 ml) a été ajoutée après 20 min et les couches séparées. La couche organique a été lavée par du NaHCO₃ saturé (100 ml), de l'eau (100 ml) et de la saumure (50 ml), puis séchée (MgSO₄), filtrée et évaporée pour donner des cristaux.
La chromatographie éclair sur colonne (éluant 0-10 % EtOAc/CH₂Cl₂) a donné 8,07 g (60 %) de produit.
RMN ¹H (CDCl₃) δ 3,50 (3H, s), 6,98 (2H, d), 7,24 (2H, d), 7,45 (2H, d), 8,08 (2H, d).

### Etape 2: 4-amino-N-(4-chlorophényl)-N-méthylbenzamide.

Une solution d'acide chlorhydrique concentré (4 ml, env. 40 mmoles) dans de l'éthanol (50 ml) et de l'eau (50 ml) a été ajoutée goutte à goutte en 2 h à une suspension au reflux de limaille de fer (6,34 g, 114 mmoles, 3 équiv) et de N-(4-chlorophényl)-N-méthyl-4-nitrobenzamide (11,0 g, 37,8 mmoles) dans de l'éthanol (200 ml) et de l'eau (200 ml).
Le mélange a été porté au reflux pendant 1 h et on l'a laissé refroidir jusqu'à la température ambiante puis basifié jusqu'à un pH d'environ 11 par de l'hydroxyde de sodium aqueux à 10 %. La suspension a été filtrée et le résidu lavé par 3 × 200 ml d'acétate d'éthyle chaud. La solution a été évaporée pour éliminer les matières organiques et le liquide aqueux extrait par EtOAc (3 × 200 ml). Les matières organiques réunies ont été lavées par de l'eau (2 × 250 ml) et de la saumure (100 ml) puis séchées (MgSO₄), filtrées et évaporées pour donner 10,4 g de solide orange. La chromatographie éclair sur colonne (éluant 10-40 % EtOAc/CH₂Cl₂) a donné 8,53 g (87 %) de produit à l'état de solide crème.
RMN ¹H (CDCl₃) δ 3,44 (3H, s), 3,81 (2H, s large), 6,44 (2H, d), 6,98 (2H, d), 7,13 (2H, d) et 7,21 (2H, d).

### Etape 3: N-[4-(N'-(4-chlorophényl)-N'-méthylaminocarbonyl)phényl]cyanoacétamide

En opérant selon un procédé analogue à celui mis en oeuvre dans l'étape 3 de la préparation de l'exemple 1, on a obtenu le composé cité ci-dessus dans un rendement de 96 %.
RMN ¹H (CDCl₃/CD₃OD) δ 3,46 (3H, s), 3,53 (2H, s), 6,99 (2H, d), 7,22 (2H, d), 7,25 (2H, d), 7,39 (2H, d).

### EXEMPLE 3: N-[4-(4'-chlorophényl-E-éthényl)phényl]-2-cyano-3-cyclopropyl-3-hydroxy-prop-2-énamide

En opérant selon un procédé analogue à celui mis en oeuvre dans l'exemple 1, on a obtenu le composé cité ci-dessus dans un rendement de 56 %.
δ (CDCl₃) 1,12-1,22 (2H, m) 1,28-1,37 (2H, m), 2,11-2,18 (1H, m), 7,03 (2H, s), 7,30-7,55 (9H, m), 15,85 (1H, s).

### Préparation de N-[4-(4'-chlorophényl-E-éthényl)phényl]cyanoacétamide

### Etape 1: E-4-chlorophényl-4'-nitrophényl-éthène.

Du sodium (2,21 g, 96,1 mmoles, 1,05 équiv) a été ajouté en gros morceaux en 1,75 h à de l'éthanol (100 ml). La solution a été agitée pendant 20 min suivant la dissolution du sodium, puis ajoutée goutte à goutte en 50 min à une solution de 4-nitrobenzylphosphonate de diéthyl (25 g, 91,5 mmoles) et de 4-chlorobenzaldéhyde (12,9 g, 91,5 mmoles) dans de l'éthanol (150 ml). Le mélange a été agité pendant 16 h puis filtré. Le solide jaune a été lavé à l'éther de pétrole (2 × 50 ml) et séché sous vide - 15,35 g (65 %) de produit.
RMN ¹H (CDCl₃), 7,11 (1H, d), 7,23 (1H, d), 7,37 (2H, d), 7,49 (2H, d), 7,63 (2H, d), 8,23 (2H, d).

### Etape 2: E-4-aminophényl-4'-chlorophényl-éthène

En opérant selon un procédé analogue à celui mis en oeuvre dans l'étape 2 de la préparation de l'exemple 2, on a obtenu le composé cité ci-dessus dans un rendement de 71 %.
δ (CDCl₃) 3,8 (2H, s large), 6,68 (2H, d), 6,85 (1H, d), 7,00 (1H, d), 7,25-7,42 (6H, m).

### Etape 3: N-[4-(4'-chlorophényl-E-éthényl)phényl]cyanoacétamide

En opérant selon un procédé analogue à celui mis en oeuvre dans l'étape 3 de la préparation de l'exemple 1, on a obtenu le composé cité ci-dessus dans un rendement de 80 %.
δ H(CDCl₃/CD₃OD) 3,59 (2H, s), 7,03 (2H, s), 7,33-7,59 (9H, m)

### EXEMPLE 4: N-{[4-(4'-chlorophényl)éthynyl]phényl}-2-cyano-3-cyclopropyl-3-hydroxy-prop-2-énamide

En opérant selon un procédé analogue à celui mis en oeuvre dans l'exemple 1, on a obtenu le composé cité ci-dessus dans un rendement de 88 %.
δ (CDCl₃) 1,12-1,20 (2H, m), 1,28-1,38 (2H, m), 2,11-2,19 (1H, m), 7,33 (2H, d), 7,46 (2H, d), 7,51 (4H, s), 7,59 (1H, s), 15,74 (1H, s).

### Préparation de N-{[4-(4'-chlorophényl)éthynyl)phényl}cyanoacétamide

### Etape 1: 1-[4-(chlorophényl)]-1,2-dibromo-2-[4'-(nitrophényl)]éthane.

Une solution de brome (14,17 g, 4,57 ml, 88,9 mmoles, 1,5 équiv) dans du chloroforme (50 ml) a été ajoutée goutte à goutte en 100 min à une solution de E-4-chlorophényl-4'-nitrophényl-éthène (15,4 g, 59,1 mmoles) dans du CHCl₃ (700 ml). La suspension a été agitée pendant 22 h puis évaporée. Le dichlorométhane (2 × 100 ml) a été évaporé du solide, pour éliminer tout brome restant, pour donner 23,4 g (95 %) de produit à l'état de solide de couleur de magnolia.
RMN ¹H (CDCl₃/CD₃OD) δ 5,40 (1H, d), 5,48 (1H, d), 7,41 (2H, d), 7,47 (2H, d), 7,69 (2H, d), 8,29 (2H, d).

### Etape 2: 4-chlorophényl-4'-nitrophényl-éthyne.

De l'hydroxyde de sodium aqueux à 50 % (150 ml) a été ajouté goutte à goutte en 1¼ h à une suspension de Bu₄NHSO₄ (68,3 g, 201 mmoles, 3 équiv) et de 1-[4-(chlorophényl)]-1,2-dibromo-2-[4'-(nitrophényl)]éthène (28,1 g, 67,0 mmoles) dans du CH₂Cl₂ (200 ml) et de l'hexane en C₆H₁₄ (200 ml). Après 30 min, de l'eau (500 ml) puis du CH₂Cl₂ (200 ml) ont été ajoutés. Le mélange a été filtré, les couches séparées et la fraction aqueuse extraite par CH₂Cl₂ (2 × 100 ml). Les matières organiques réunies ont été lavées par de l'eau (4 × 250 ml), de la saumure (100 ml) puis séchées (MgSO₄) filtrées et évaporées pour donner 29,9 g de solide vert foncé.
La chromatographie éclair sur colonne (5 --→ 100 % EtOAc/40-60 éther de pétrole puis --→ 60 % acétone/EtOAc) a donné 16,29 g (94 %) de produit.
RMN ¹H δ (CDCl₃) 7,37 (2H, d), 7,50 (2H, d), 7,67 (2H, d), 8,23 (2H, d).

### Etape 3: 4-aminophényl-4'-chlorophényl-éthyne

En opérant selon un procédé analogue à celui mis en oeuvre dans l'étape 2 de la préparation de l'exemple 2, on a obtenu le composé cité ci-dessus dans un rendement de 70 %.
δ (CDCl₃) 3,84 (2H, s large), 6,64 (2H, d), 7,26-7,44 (6H, m).

### Etape 4: N-{[4-(4'-chlorophényl)éthynyl]phényl}cyanoacétamide

En opérant selon un procédé analogue à celui mis en oeuvre dans l'étape 3 de la préparation de l'exemple 1, on a obtenu le composé cité ci-dessus dans un rendement de 93 %.
δ H(d₆-DMSO) 3,99 (2H, s), 7,51-7,70 (8H, m), 10,56 (1H, s).

### EXEMPLE 5: N-[4'-(4-chlorophényl-Z-éthényl)phényl]-2-cyano-3-cyclopropyl-3-hydroxy-prop-2-énamide

En opérant selon un procédé analogue à celui mis en oeuvre dans l'exemple 1, on a obtenu le composé cité ci-dessus dans un rendement de 84 %.
δ H(d₆-DMSO) 0,96-1,02 (4H, m), 2,14-2,24 (1H, m), 6,57 (1H, d), 6,66 (1H, d), 7,18 (2H, d), 7,29 (2H, d), 7,38 (2H, d), 7,47 (2H, d), 11,06 (1H, s).

### Préparation de N-{[4-(4-chlorophényl-Z-éthényl)phényl]cyano-acétamide

### Etape 1: Z-4-chlorophényl-4'-nitrophényl-éthène.

Un mélange de 4-chlorophényl-4'-nitrophényl-éthyne (9,0 g, 34,9 mmoles), de palladium sur carbonate de calcium empoisonné par du plomb (catalyseur hétérogène, 900 mg) et de quinoléine (902 mg, 825 µl, 7,00 moles, 0,2 équiv) a été agité sous hydrogène dans de l'acétate d'éthyle (1 l) jusqu'à ce que la consommation d'hydrogène se soit arrêtée. Le mélange a été filtré et évaporé. La RMN ¹H a indiqué un mélange de matière de départ et de produit. Ainsi, le mélange réactionnel a été dissous dans EtoAc (175 ml) et on a ajouté de la quinoléine (1 ml) et du catalyseur hétérogène (1,5 g) et on a repris l'hydrogénation. Lorsque la consommation d'hydrogène s'est arrêtée, le mélange a été filtré, évaporé et purifié par chromatographie éclair sur colonne (éluant 5-20 % EtOAc/40-60 éther de pétrole) pour donner 7,98 g (88 %) de produit.
RMN ¹H (CDCl₃) δ 6,63 (1H, d), 6,75 (1H, d), 7,12 (2H, d), 7,23 (2H, d), 7,36 (2H, d), 8,09 (2H, d).

### Etape 2: Z-4-aminophényl-4'-chlorophényl-éthène

En opérant selon un procédé analogue à celui mis en oeuvre dans l'étape 2 de la préparation de l'exemple 2, on a obtenu le composé cité ci-dessus dans un rendement de 53 %.
δ (CDCl₃) 3,69 (2H, s large), 6,35 (1H, d), 6,50 (1H, d), 6,54 (2H, d), 7,04 (2H, d), 7,20 (4H, s).

### Etape 3: N-[4-(4'-chlorophényl-Z-éthényl)phényl]cyanoacétamide

En opérant selon un procédé analogue à celui mis en oeuvre dans l'étape 3 de la préparation de l'exemple 1, on a obtenu le composé cité ci-dessus dans un rendement de 86 %.
δ H(d₆-DMSO) 3,94 (2H, s), 6,59 (1H, d), 6,68 (1H, d), 7,23 (2H, d), 7,27 (2H, d), 7,38 (2H, d), 7,50 (2H, d).

### EXEMPLE 6 N-[4-(4'-fluorophényl-E-éthényl)phényl]-2-cyano-3-cyclopropyl-3-hydroxy-prop-2-énamide

En opérant selon un procédé analogue à celui mis en oeuvre dans l'exemple 1, on a obtenu le composé cité ci-dessus dans un rendement de 54 %.
δ H(d₆-DMSO) 1,07-1,13 (4H, m), 2,13-2,28 (1H, m), 7,14-7,44 (4H, m), 7,59-7,75 (6H, m), 10,69 (1H, s).

### Préparation de N-[4-(4'-fluorophényl-E-éthényl)phényl]cyanoacétamide

### Etape 1: E-4-fluorophényl-4'-nitrophényl-éthène.

En opérant selon un procédé analogue à celui mis en oeuvre dans l'étape 1 de la préparation de l'exemple 3, on a obtenu le composé cité ci-dessus dans un rendement de 69 %.
δ 7,01-7,28 (4H, m), 7,53 (2H, dd), 7,62 (2H, d), 8,23 (2H, d).

### Etape 2: E-4-aminophényl-4'-fluorophényl-éthène

En opérant selon un procédé analogue à celui mis en oeuvre dans l'étape 2 de la préparation de l'exemple 2, on a obtenu le composé cité ci-dessus dans un rendement de 81 %.
δ (CDCl₃) 3,75 (2H, s large), 6,67 (2H, d), 6,90 (2H, m), 7,02 (2H, t), 7,32 (2H, d), 7,43 (2H, dd).

### Etape 3: N-[4-(4'-fluorophényl-E-éthényl)phényl]cyanoacétamide

En opérant selon un procédé analogue à celui mis en oeuvre dans l'étape 3 de la préparation de l'exemple 1, on a obtenu le composé cité ci-dessus dans un rendement de 91 %.
δ H(d₆-DMSO) 3,97 (2H, s), 7,22 (2H, s), 7,27 (2H, d), 7,61-7,71 (6H, m).

### EXEMPLE 7: N-[4-(4'-fluorophényléthynyl)phényl]-2-cyano-3-cyclopropyl-3-hydroxy-prop-2-énamide

En opérant selon un procédé analogue à celui mis en oeuvre dans l'exemple 1, on a obtenu le composé cité ci-dessus dans un rendement de 84 %.
δ H(d₆-DMSO) 1,10-1,17 (4H, m), 2,16-2,25 (1H, m), 7,31 (2H, t), 7,46-7,69 (7H, m), 10,74 (1H, s).

### Préparation de N-{4-[(4'-fluorophényl)éthynyl]phényl}cyanoacétamide

### Etape 1: 1,2-dibromo-1-(4-fluorophényl)-2-(4'-nitrophényl)éthane

En opérant selon un procédé analogue à celui mis en oeuvre dans l'étape 1 de la préparation de l'exemple 4, on a obtenu le composé cité ci-dessus dans un rendement de 93 %.
δ H 5,42 (2H, s), 7,12 (2H, t), 7,50 (2H, dd), 7,67 (2H, d), 8,28 (2H, d).

### Etape 2: 4-fluorophényl-4'-nitrophényl-éthyne

En opérant selon un procédé analogue à celui mis en oeuvre dans l'étape 2 de la préparation de l'exemple 4, on a obtenu le composé cité ci-dessus dans un rendement de 91 %.
RMN ¹H (CDCl₃) 7,09 (2H, t), 7,55 (2H, dd), 7,65 (2H, d), 8,55 (2H, d).

### Etape 3: 4-aminophényl-4'-fluorophényl-éthyne

En opérant selon un procédé analogue à celui mis en oeuvre dans l'étape 2 de la préparation de l'exemple 2, on a obtenu le composé cité ci-dessus dans un rendement de 71 %.
δ (CDCl₃) 3,83 (2H, s large), 6,65 (2H, d), 7,03 (2H, t), 7,34 (2H, d), 7,48 (2H, dd).

### Etape 4: N-{4-[(4'-fluorophényl)éthynyl]phényl}cyanoacétamide

En opérant selon un procédé analogue à celui mis en En opérant selon un procédé analogue à celui mis en oeuvre dans l'étape 3 de la préparation de l'exemple 1, on a obtenu le composé cité ci-dessus dans un rendement de 93 %.
δ H(d₆-DMSO) 3,99 (2H, s), 7,31 (2H, t), 7,54-7,68 (6H, m), 10,56 (1H, s).

### EXEMPLE 8: N-[4-(phényléthyl)phényl]-2-cyano-3-cyclopropyl-3-hydroxy-prop-2-énamide

En opérant selon un procédé analogue à celui mis en oeuvre dans l'exemple 1, on a obtenu le composé cité ci-dessus dans un rendement de 28 %.
δ H(d₆-DMSO) 1,15-1,26 (4H, m), 2,18-2,24 (1H, m), 2,90 (4H, s), 7,18-7,36 (7H, m), 7,46 (2H, d), 10,24 (1H, s).

### Préparation de N-{4-[2-(phényléthyl)]phényl}cyanoacétamide

### Etape 1: E-4-(nitrophényl)-2-phényléthène

En opérant selon un procédé analogue à celui mis en oeuvre dans l'étape 1 de la préparation de l'exemple 3, on a obtenu le composé cité ci-dessus dans un rendement de 76 % - mélange ≃ 3:1 des isomères trans:cis.

### Etape 2: 1-(4-Aminophényl)-2-phényléthane.

Un mélange de E-4-(nitrophényl)-2-phényléthène (14,7 g. 65,4 mmoles) et d'oxyde de platine (82 mg, 327 µmoles, 5 % molaire) a été agité sous hydrogène dans de l'éthanol (500 ml) pendant 19 h. La suspension a été filtrée sur célite et évaporée pour donner 14,7 g (> 100 %) de produit à l'état de cristaux jaunes.
δ H (CDCl₃) 2,76-2,87 (4H, m), 3,53 (2H, s large), 6,61 (2H, d), 6,96 (2H, d), 7,13-7,31 (5H, m).

### Etape 3: N-[4-[2(phényléthyl)]phényl]cyanoacétamide

En opérant selon un procédé analogue à celui mis en oeuvre dans l'exemple 1, on a obtenu le composé cité ci-dessus dans un rendement de 80 %.
δ H(d₆-DMSO) 2,88 (4H, s), 3,91 (2H, s), 7,19-7,31 (7H, m), 7,48 (2H, d), 10,32 (1H, s).

### EXEMPLE 9: N-[4-biphénylyl]-2-cyano-3-cyclopropyl-3-hydroxyprop-2-énamide

En opérant selon un procédé analogue à celui mis en oeuvre dans l'exemple 1, on a obtenu le composé cité ci-dessus dans un rendement de 67 %.
δ H (CDCl₃) 1,11-1,24 (2H, m), 1,27-1,43 (2H, m), 2,12-2,20 (1H, m), 7,26-7,63 (10H, m), 15,89 (1H, s).

### Préparation de N-[4-biphénylyl]cyanoacétamide

### Etape 1: 4-nitrobiphényl.

1-Bromo-4-nitrobenzène (12,50 g, 61,9 mmoles) a été ajouté à tétrakis(triphénylphosphine)palladium (358 mg, 310 µmoles, 0,5 % molaire) dans du diméthoxyéthane (200 ml). Après agitation pendant 10 min, on a ajouté de l'acide phénylboronique (11,3 g, 92,8 mmoles, 1,5 équiv) et de l'éthanol (100 ml). Après encore 10 min, on a ajouté du carbonate de sodium aqueux à 2M (263 ml, 526 mmoles, 8,5 équiv) et la suspension a été chauffée au reflux pendant 20 min puis on a laissé refroidir jusqu'à la température ambiante. Le mélange a été repris par EtOAc (250 ml) et H₂O (200 ml). La couche aqueuse a été extraite par EtOAc (200 ml) et les matières organiques réunies ont été lavées par de l'eau (2 × 250 ml) et de la saumure (100 ml), séchées (MgSO₄), filtrées et évaporées pour donner 15,7 g de solide. La chromatographie éclair sur colonne (2,5-15 % EtOAc/40-60 éther de pétrole) a donné 11,8 g (96 %) de produit à l'état de solide jaune pâle.
RMN ¹H δ H (CDCl₃) 7,43-7,55 (3H, m), 7,58-7,65 (2H, m), 7,73 (2H, d), 8,29 (2H, d).

### Etape 2: 4-aminobiphényle

En opérant selon un procédé analogue à celui mis en oeuvre dans l'étape 2 de la préparation de l'exemple 8, on a obtenu le composé cité ci-dessus.
RMN ¹H δ (CDCl₃) 3,72 (2H, s large), 6,76 (2H, d), 7,25-7,28 (1H, m), 7,35-7,45 (4H, m), 7,51-7,57 (2H, m).

### Etape 3: N-[4-biphénylyl]cyanoacétamide

En opérant selon un procédé analogue à celui mis en oeuvre dans l'étape 3 de la préparation de l'exemple 1, on a obtenu le composé cité ci-dessus dans un rendement de 72 %.
δ H (d₆-DMSO) 3,99 (2H, s), 7,37-7,54 (3H, m), 7,67-7,73 (6H, m), 10,46 (1H, s).

### EXEMPLE 10 : N-[4-(4'-fluorophényl)phényl]-2-cyano-3-cyclopropyl-3-hydroxy-prop-2-énamide

En opérant selon un procédé analogue à celui mis en oeuvre dans l'exemple 1, on a obtenu le composé cité ci-dessus dans un rendement de 65 %.
δ H (d₆-DMSO) 1,02-1,08 (4H, m), 2,16-2,28 (1H, m), 7,32 (2H, dd), 7,64-7,77 (6H, m), 10,97 (1H, s large).

### Préparation de N-[4-(4'-fluorophényl)phényl]cyanoacétamide

En opérant selon un procédé analogue à celui mis en oeuvre dans l'étape 3 de la préparation de l'exemple 1, on a obtenu le composé cité ci-dessus dans un rendement de 91 %.
δ H (d₆-DMSO) 3,99 (2H, s), 7,32 (2H, dd), 7,68-7,77 (6H, m), 10,56 (1H, s).

### EXEMPLE 11: N-[4-(4'-chlorophénylméthyl)phényl]-2-cyano-3-cyclopropyl-3-hydroxy-prop-2-énamide

En opérant selon un procédé analogue à celui mis en oeuvre dans l'exemple 1, on a obtenu le composé cité ci-dessus dans un rendement de 83 %.
δ H (CDCl₃) 1,11-1,22 (2H, m), 1,25-1,35 (2H, m), 2,10-2,19 (1H, m), 3,93 (2H, s), 7,00-7,40 (8H, m), 7,47 (1H, s), 15,87 (1H, s).

### Préparation de N-[4-(4'-chlorophénylméthyl)phényl]cyanoacétamide.

### Etape 1: 4-chloro-4'-nitrobenzophénone

Préparé comme décrit à l'étape 1 de l'exemple 1.

### Etape 2: 4-amino-4'-chlorobenzophénone

Préparé comme décrit à l'étape 2 de l'exemple 1.

### Etape 3: 4-(4'-chlorophénylméthyl)aniline

Une suspension de 4-amino-4'-chlorobenzophénone (4,27 g, 18,4 mmoles) et d'amalgame de zinc (8,54 g) dans du toluène (80 ml) contenant de l'eau (4 ml) a été chauffée au reflux. De l'acide chlorhydrique concentré (6 ml) a été ajouté en 1,5 h. Après 22,5 h, on a laissé le mélange se refroidir jusqu'à la température ambiante et on a ajouté de l'acétate d'éthyle (100 ml) et de l'eau (100 ml). Les couches ont été séparées et la couche aqueuse extraite par de l'acétate d'éthyl (50 ml). Les matières organiques réunies ont été lavées par de l'eau (2 × 100 ml) et de la saumure (50 ml), séchées (MgSO₄), filtrées et évaporées pour donner 3,03 g d'huile orange. La chromatographie éclair (éluant 20-35 % acétate d'éthyl/éther de pétrole) a donné le produit cité ci-dessus à l'état de liquide orange, 915 mg (24,6 % de rendement).
RMN ¹H (CDCl₃) δ 3,58 (2H, s large), 3,82 (2H, s), 6,62 (2H, d), 6,94 (2H, d), 7,08 (2H, d), 7,22 (2H, d).

### Etape 4: N-[4-(4'-chlorophénylméthyl)phényl]cyanoacétamide

En partant de 4-(4'-chlorophénylméthyl)aniline et en opérant comme à l'étape 3 de l'exemple 1, on a obtenu le composé cité ci-dessus dans un rendement de 83 %.
RMN ¹H (d₆-DMSO) δ 3,92 (4H, s), 7,23 (2H, d), 7,28 (2H, d), 7,39 (2H, d), 7,50 (2H, d), 10,32 (1H, s).

### EXEMPLE 12: N-{4-[2-(4'-chlorophényl)éthyl]phényl}-2-cyano-3-cyclopropyl-3-hydroxy-prop-2-énamide

En opérant selon un procédé analogue à celui mis en oeuvre dans l'exemple 1, on a obtenu le composé cité ci-dessus.

Les données spectrales, les rendements, les points de fusion et les données analytiques pour les exemples sont donnés au tableau I.

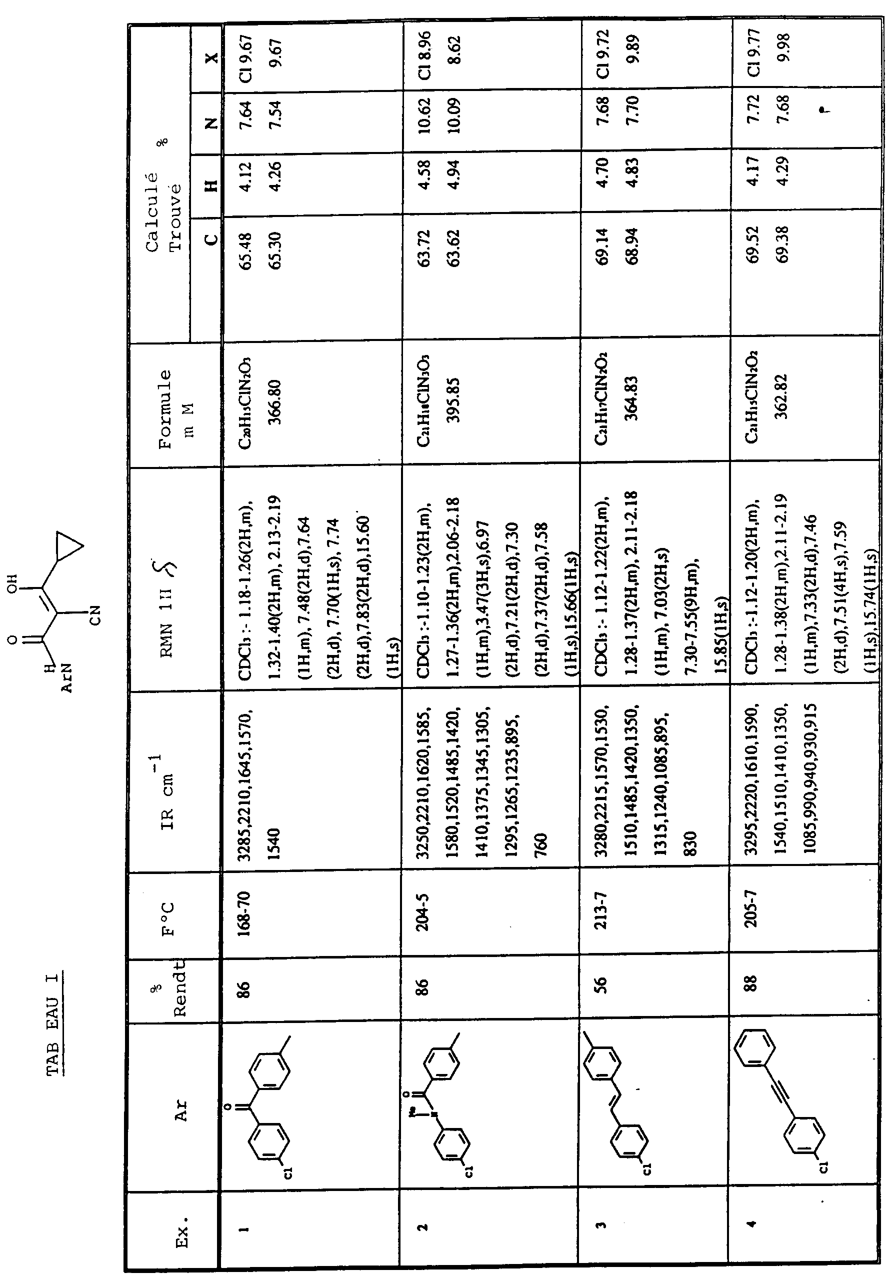

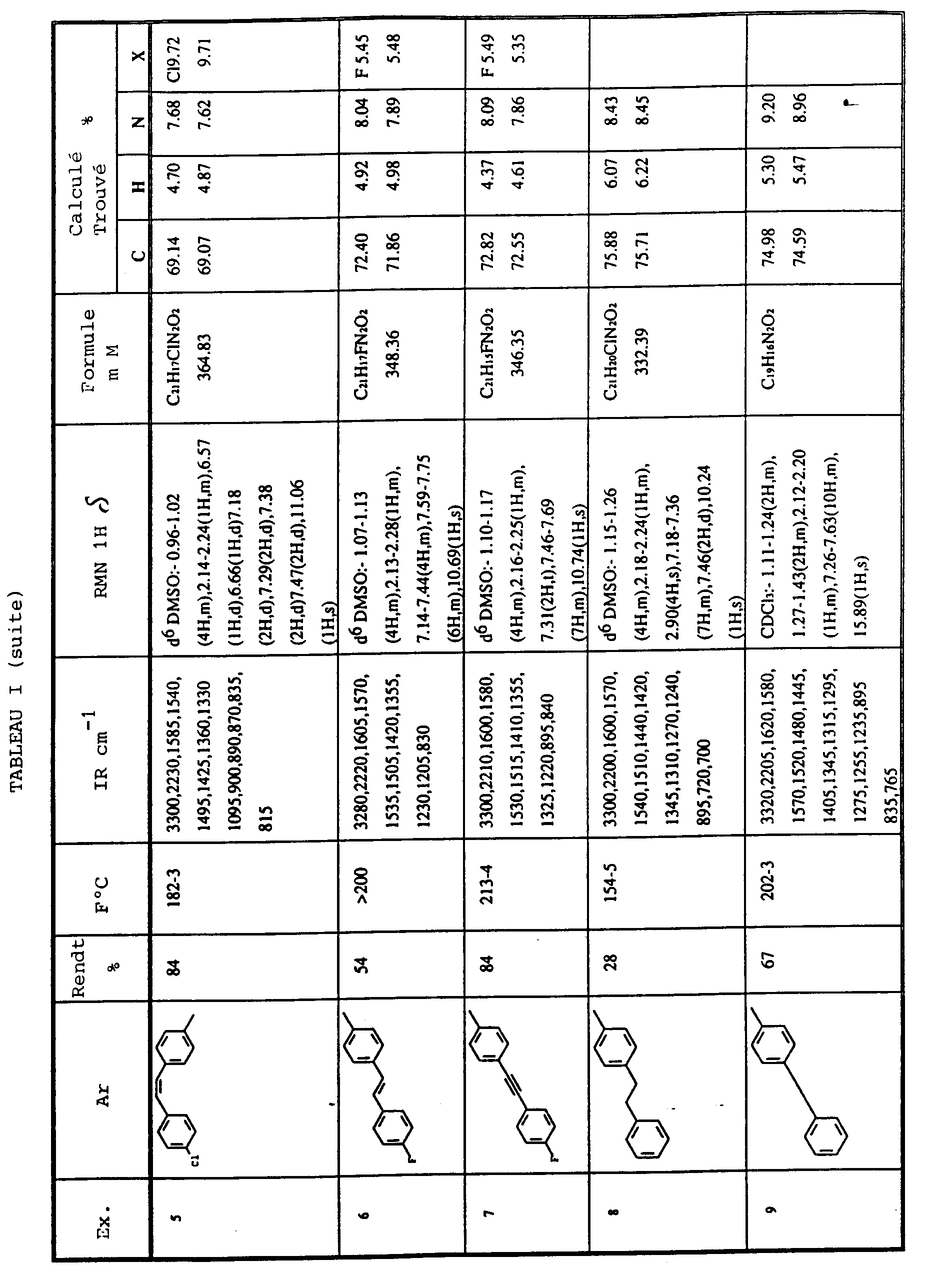

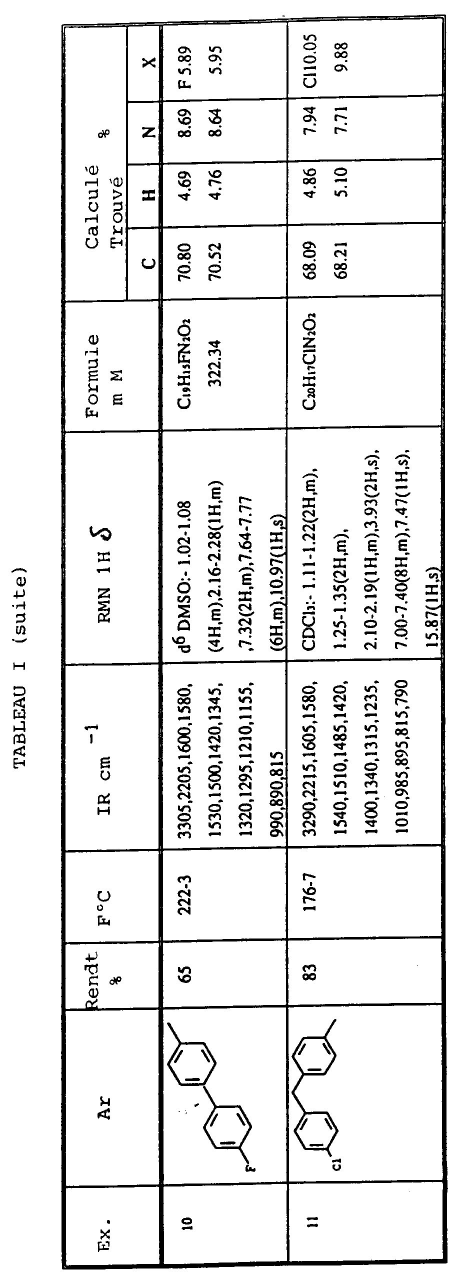

### EXEMPLE 13 :

On a préparé des comprimés répondant à la formule suivante :

| | |
|---|---|
| - Composé de l'exemple 1 | 20 mg |
| - Excipient q.s.p. un comprimé terminé à | 150 mg |
| (Détail de l'excipient : lactose, amidon, talc, stéarate de magnésium). | |

### EXEMPLE 14 :

On a préparé des comprimés répondant à la formule suivante :

| | |
|---|---|
| - Composé de l'exemple 2 | 20 mg |
| - Excipient q.s.p. un comprimé terminé à | 150 mg |
| (Détail de l'excipient : lactose, amidon, talc, stéarate de magnésium). | |

### ACTIVITE PHARMACOLOGIQUE

### Méthodes de tests biochimiques.

### 1er test

Oedème de la patte de rat (PO-R) induit par de la carragénine.

Une heure après l'administration orale des composés du test ou du véhicule témoin à des groupes de rats (n = 6-12, CFHB mâles, d'un poids entre 160-180 g), on injecte 1 mg de carragénine dissoute dans 0,2 ml de solution saline dans le coussinet digital de la patte arrière droite. Les pattes contralatérales reçoivent les injections témoin de solution saline. Les réactions d'oedèmes des pattes sont évaluées trois heures plus tard.

### 2ème test

Oedème à hypersensibilité de type retard de la patte de souris (DTH-M).

Des groupes de souris (n = 8-10), mâles CD-1, d'un poids entre 25-30 g) sont sensibilisés par une injection sous-cutanée de 1 mg de sérum-albumine bovine méthylé (MBSA) dans des volumes de 0,2 ml d'une solution saline/émulsion d'adjuvant complet de Freund (FCA). Des groupes témoin négatifs reçoivent des injections de solution saline/émulsion de FCA. Les réactions DHT d'oedème de la patte sont évaluées 24 heures après le défi dans le coussinet digital de la patte arrière droite avec 0,1 mg de MBSA dans des volumes de 0,05 ml de solution saline le 7ème jour après la sensibilisation. Les pattes contralatérales reçoivent des injections témoin de solution saline. Les composés du test ou les véhicules témoin sont administrés oralement une fois par jour les 4ème, 5ème et 6ème jours et deux fois par jour le 7ème jour, 1 heure avant et 6 heures après le défi au MBSA.

### 3ème test

Oedème à hypersensibilité de type retard de la patte de rat (DTH-R).

Des groupes de rats (n = 8-12, mâles CFHB, d'un poids entre 160-180 g) sont sensibilisés par une injection sous-cutanée à la base de la queue avec des volumes de 0,1 ml de FCA. Des groupes témoin négatifs reçoivent une injection d'adjuvant incomplet de Freund. Les réactions DTH d'oedème de la patte sont évaluées 24 heures après le défi, dans le coussinet digital de la patte arrière droite avec 0,4 mg d'un extrait antigène de Mycobacterium tuberculosis dans des volumes de 0,2 ml de solution saline le 7ème jour après la sensibilisation. Les pattes contralatérales reçoivent des injections témoin de solution saline.

Les composés du test sont administrés oralement une fois par jour les 4ème, 5ème et 6ème jour et deux fois par jour le 7ème jour, 1 heure avant et 6 heures après le défi antigénique.

Les résultats de ces tests sont donnés au tableau II.

Les doses sont données en unités de mg/kg p.o.

**TABLEAU II**

| Exemple | Test 1 | | Test 2 | | Test 3 | |
|---|---|---|---|---|---|---|
| | % | Dose | % | Dose | % | Dose |
| | inhibition | | inhibition | | inhibition | |
| 1 | 28 | (50) | 4 | (30) | -19 | (10) |
| 2 | 34 | (50) | -8 | (100) | 22 | (50) |
| 3 | -8 | (50) | 69 | (100) | 48 | (50) |
| 4 | 13 | (50) | 61 | (30) | 61 | (10) |
| 5 | -7 | (50) | 19 | (100) | 52 | (50) |
| 6 | -4 | (10) | 76 | (30) | 47 | (10) |
| 7 | 41 | (50) | 19 | (10) | 33 | (10) |
| 8 | 22 | (50) | 5 | (100) | 51 | (50) |
| 9 | 35 | (50) | 33 | (100) | 70 | (50) |
| 10 | -5 | (10) | 53 | (30) | 79 | (10) |
| 11 | 17 | (50) | 23 | (30) | - | - |

## Revendications

1. N-(phenyl)-2-cyano-3-hydroxy-propenamides répondant à la formule générale (I) : 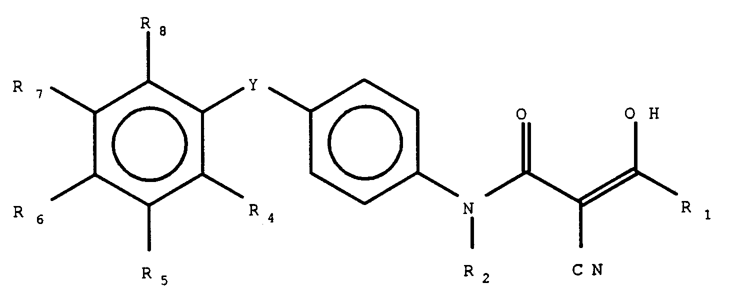 dans laquelle :
- R₁ représente un radical cycloalkyle renfermant de 3 à 6 atomes de carbone,
- R₂ représente un atome d'hydrogène, un radical alkyle renfermant de 1 à 3 atomes de carbone,
- Y représente un groupement:
-NR₃- , -SO₂-NR₃- , -NR₃-SO₂- , -CHOH- , -CF₂- , -(CH₂)ₘ- , 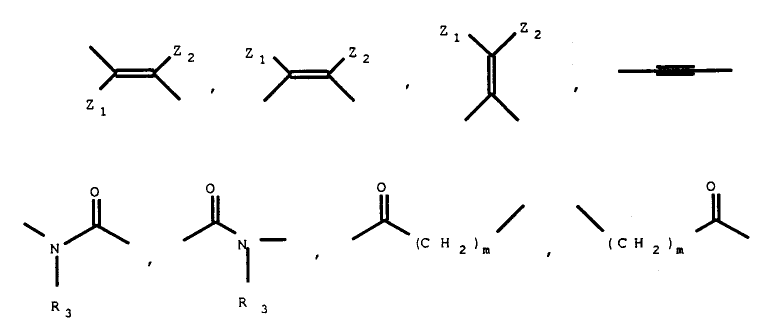 groupements dans lesquels Z₁ et Z₂, identiques ou différents, représentent un atome d'hydrogène, un atome d'halogène, un radical alkyle renfermant de 1 à 3 atomes de carbone, un groupement -CN un groupement - NO₂ , R₃ représente un atome d'hydrogène ou un radical alkyle renfermant de 1 à 3 atomes de carbone, m est un nombre entier qui peut varier de 0 à 6 ,
- R₄, R₅, R₆, R₇ et R₈, identiques ou différents, représentent un atome d'hydrogène, un atome d'halogène, un groupement - CN, un groupement - NO₂, un radical alkyle, linéaire ou ramifié, renfermant de 1 à 6 atomes de carbone, un radical cycloalkyle renfermant de 3 à 6 atomes de carbone, un radical alcoxy, linéaire ou ramifié, renfermant de 1 à 6 atomes de carbone, un radical alkylthio renfermant de 1 à 6 atomes de carbone, un radical -(CH₂)ₙ-CX₃ , un radical
-O-(CH₂)ₙ-CX₃ , un radical -S-(CH₂)ₙ-CX₃ , radicaux dans lesquels n représente un nombre entier compris entre 0 et 3 et X représente un atome d'halogène, ou un groupement - CO - R₁₀ dans lequel R₁₀ représente un atome d'hydrogène, un radical alkyle, linéaire ou ramifié, renfermant de 1 à 6 atomes de carbone, un radical cycloalkyle renfermant de 3 à 6 atomes de carbone,
ou R₆ et R₇ , forment ensemble un groupement -O-CH₂-O- , et R₄, R₅ et R₈ ont la définition indiquée ci-dessus, leurs formes tautomères, ainsi que leurs sels d'addition avec les bases minérales ou organiques.

2. N-(phenyl) 2-cyano-3-hydroxy-propenamides, tels que définis par la formule (I) de la revendication 1, caractérisés en ce que dans ladite formule (I),
R₁ représente un radical cyclopropyle,
Y, R₂, R₄, R₅, R₆, R₇ et R₈ ont la signification déjà indiquée, leurs formes tautomères, ainsi que leurs sels d'addition avec les bases minérales ou organiques.

3. N-(phenyl) 2-cyano-3-hydroxy-propenamides, répondant à la formule (I) selon la revendication 1 ou 2, caractérisés en ce que dans ladite formule (I),
R₂ représente un atome d'hydrogène ou un radical méthyle et R₁,
Y, R₄, R₅, R₆, R₇ et R₈ ont la signification déjà indiquée, leurs formes tautomères ainsi que leurs sels d'addition avec les bases minérales ou organiques.

4. N-(phenyl) 2-cyano-3-hydroxy-propenamides répondant à la formule (I) selon l'une quelconque des revendications 1, 2 et 3, caractérisés en ce que dans ladite formule (I),
R₁ représente un radical cyclopropyle,
R₂ représente un atome d'hydrogène ou un radical méthyle,
Y représente un groupement -(CH₂)ₘ- dans lequel m est égal à 0, 1 ou 2 ou un groupement: 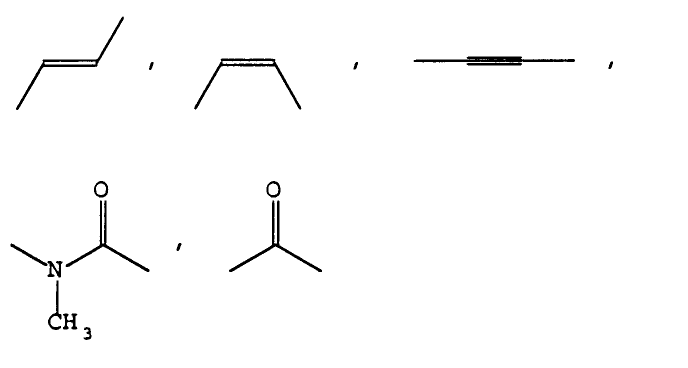 et soit R₄, R₅, R₆, R₇ et R₈ , identiques ou différents, représentent un atome d'hydrogène, un atome de chlore, de brome ou de fluor, un groupement - CN , un groupement - NO₂ , un radical méthyle, un radical cyclopropyle, un radical méthoxy, un radical méthylthio, un radical
-(CH₂)ₙ-CF₃ , un radical -O-(CH₂)ₙ-CF₃ , un radical -S-(CH₂)ₙ-CF₃, radicaux dans lesquels n représente un nombre entier compris entre 0 et 3, ou un groupement - CO - R₁₀ dans lequel R₁₀ représente un atome d'hydrogène, un radical méthyle, un radical cyclopropyle, soit R₆ et R₇ , forment ensemble un groupement 6,7-O-CH₂-O- et R₄, R₅ et R₈ ont la signification indiquée à la revendication 1, leurs formes tautomères, ainsi que leurs sels d'addition avec les bases minérales ou organiques.

5. N-phenyl 2-cyano-3-hydroxy-propenamides répondant à la formule (I) selon l'une quelconque des revendications 1, 2, 3 et 4, caractérisés en ce que dans ladite formule (I),
R₁ représente un radical cyclopropyle, R₂ représente un atome d'hydrogène ou un radical méthyle,
Y représente un groupement -(CH₂)ₘ- dans lequel m est égal à 0, 1 ou 2 ou un groupement : 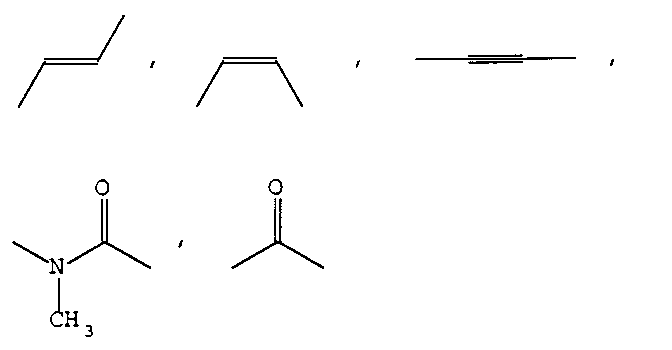
R₄, R₅, R₆, R₇ et R₈ , identiques ou différents, représentent un atome d'hydrogène, un atome de chlore, de brome ou de fluor, un groupement - NO₂ , un radical méthyle ou un radical -CF₃ , leurs formes tautomères, ainsi que leurs sels d'addition avec les bases minérales ou organiques.

6. N-phenyl 2-cyano-3-hydroxy-propenamides répondant à la formule (I) selon l'une quelconque des revendications 1 à 5 dont les noms suivent :
- N-[4-(4'-chlorophenyl-E-ethenyl) phenyl] 2-cyano 3-cyclopropyl 3-hydroxy-prop-2-enamide,
- N-[4-(4'-fluorophenyl-E-ethenyl) phenyl] 2-cyano 3-cyclopropyl 3-hydroxy-prop-2-enamide,
- N-[4-biphenylyl] 2-cyano 3-cyclopropyl 3-hydroxy-prop-2-enamide,
- N-[4-(4'-fluorophenyl) phenyl] 2-cyano 3-cyclopropyl 3-hydroxy-prop-2-enamide, ainsi que leurs sels d'addition avec les bases minérales ou organiques.

7. Procédé de préparation des N-phenyl 2-cyano-3-hydroxy-propenamides tels que définis par la formule (I) de la revendication 1, ainsi que de leurs sels, caractérisé en ce que l'on fait réagir un produit de formule (II) : 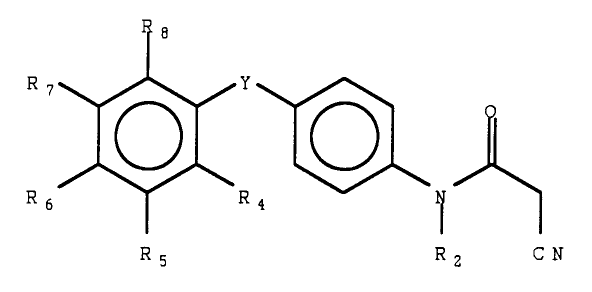 dans laquelle R₂, Y, R₄, R₅, R₆, R₇ et R₈, ont la signification déjà indiquée à la revendication 1, avec successivement de l'hydrure de sodium, si nécessaire en présence d'un catalyseur, puis fait réagir le produit avec le dérivé fonctionnel d'un acide de formule (III): 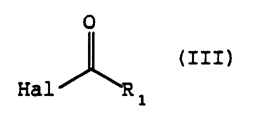 dans laquelle Hal représente un atome d'halogène et R₁ a la signification déjà indiquée à la revendication 1, pour obtenir un produit de formule (I) correspondant que l'on isole et si désiré salifie ou bien avec le dérivé fonctionnel d'un acide de formule (III_{A}) : 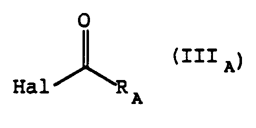 dans laquelle Hal représente un atome d'halogène et R_{A} représente un radical R₁ dûment protégé, pour obtenir un produit de formule (I_{A}): 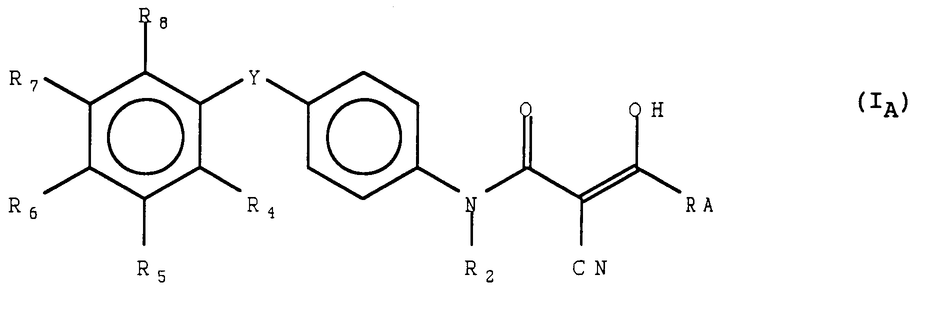 dans laquelle R_{A}, R₂, Y, R₄, R₅, R₆, R₇ et R₈ ont la signification déjà indiquée, déprotège le produit de formule (I_{A}) ainsi obtenu dans lequel R_{A} a la signification déjà indiquée, pour obtenir un produit de formule (I) correspondant, que l'on isole et si désiré salifie.

8. Procédé de préparation selon la revendication 7, caractérisé en ce que la réaction du produit de formule (II) avec l'hydrure de sodium est effectuée en présence d'imidazole, au sein d'un solvant organique anhydre tel que le tétrahydrofuranne ou le dichloromethane.

9. Procédé de préparation selon la revendication 7 ou 8, caractérisé en ce que la réaction avec le dérivé fonctionnel de l'acide de formule (III) ou (III_{A}) est effectuée à basse température ou à température ambiante, en présence d'imidazole au sein d'un solvant organique anhydre tel que le tétrahydrofuranne ou le dichlorométhane.

10. Procédé de préparation selon l'une quelconque des revendications 7 à 9, caractérisé en ce que dans le composé de formule (III_{A}), R_{A} représente un radical R₁ protégé par un groupement arylséléno, de préférence un radical phénylséléno.

11. Procédé de préparation selon l'une quelconque des revendications 7 à 10, caractérisé en ce que le produit de formule (II) est préparé par réaction d'un produit de formule (IV) : 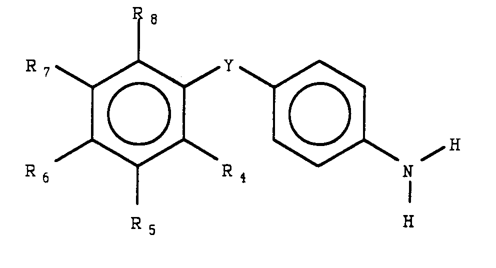 dans laquelle Y, R₄, R₅, R₆, R₇ et R₈ ont la signification indiquée à la revendication 7 avec l'acide cyanoacétique.

12. Procédé de préparation selon la revendication 11, caractérisé en ce que la réaction du composé de formule (IV) avec l'acide cyanoacétique est effectuée en présence de dicyclocarbodiimide ou de pentachlorure de phosphore au sein d'un solvant organique anhydre tel que le tétrahydrofuranne ou le dichlorométhane.

13. Procédé de préparation selon la revendication 11 ou 12, caractérisé en ce que le composé de formule (IV) est préparé par réduction du produit nitré correspondant de formule (V): 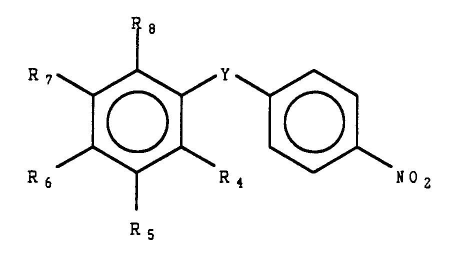 dans laquelle Y, R₄, R₅, R₆, R₇ et R₈ ont la signification indiquée à la revendication 11.

14. Médicaments, caractérisés en ce qu'ils sont constitués par les N-phenyl 2-cyano-3-hydroxy-propenamides tels que définis par la formule (I) de la revendication 1, ainsi que par leurs sels d'addition avec les bases pharmaceutiquement acceptables.

15. Médicaments, caractérisés en ce qu'ils sont constitués par les N-phenyl 2-cyano-3-hydroxy-propenamides tels que définis à l'une quelconque des revendications 2 à 6, ainsi que par leurs sels d'addition avec les bases pharmaceutiquement acceptables.

16. Compositions pharmaceutiques, caractérisées en ce qu'elles renferment à titre de principe actif, l'un au moins des médicaments tels que définis à l'une quelconque des revendications 14 et 15.

## Claims

1. N-(phenyl)-2-cyano-3-hydroxy-propenamides corresponding to general formula (I): 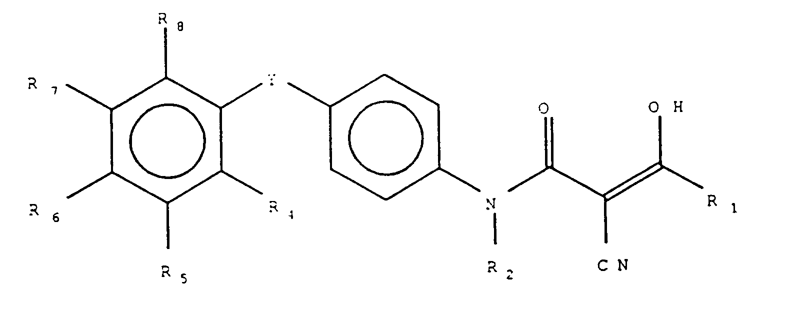 in which:
- R₁ represents a cycloalkl radical containing 3 to 6 carbon atoms,
- R₂ represents a hydrogen atom, an alkyl radical containing from 1 to 3 carbon atoms,
- Y represents a group:
-NR₃- , -SO₂-NR₃- , -NR₃-SO₂- , -CHOH- , -CF₂- , -(CH₂)ₘ-, 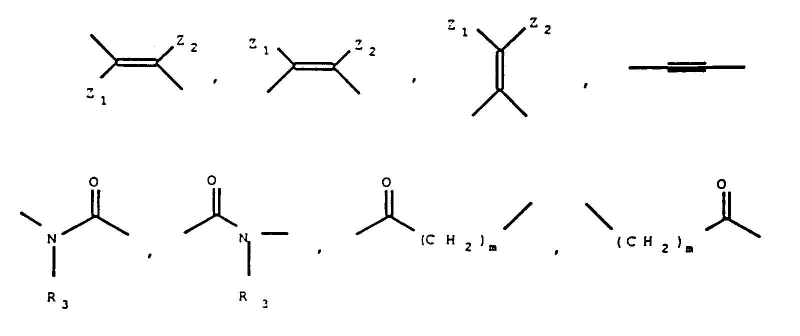 groups in which Z₁ and Z₂, identical or different, represent a hydrogen atom, a halogen atom, an alkyl radical containing from 1 to 3 carbon atoms, a -CN group, an NO₂ group, R₃ represents a hydrogen atom or an alkyl radical containing from 1 to 3 carbon atoms, m is an integer which can vary from 0 to 6,
- R₄, R₅, R₆, R₇ and R₈, which may be identical or different, represent a hydrogen atom, a halogen atom, a -CN group, an - NO₂ group, a linear or branched alkyl radical containing from 1 to 6 carbon atoms, a cycloalkyl radical containing from 3 to 6 carbon atoms, a linear or branched alcoxy radical containing from 1 to 6 carbon atoms, an alkylthio radical containing from 1 to 6 carbon atoms, a -(CH₂)ₙ-CX₃ radical, an -O-(CH₂)ₙ-CX₃ radical a -S-(CH₂)ₙ-CX₃ radical, in which radicals n represent an integer comprised between 0 and 3 and X represents a halogen atom, or a - CO - R₁₀ group in which R₁₀ represents a hydrogen atom, a linear or branched alkyl radical containing from 1 to 6 carbon atoms, a cycloalkyl containing from 3 to 6 carbon atoms,
or R₆ and R₇ together form a -O-CH₂-O- group, and R₄, R₅ and R₈ have the definition indicated above, their tautomeric forms, and their addition salts with mineral or organic bases.

2. N-(phenyl) 2-cyano-3-hydroxy-propenamides, as defined in formula (I) of claim 1, characterized in that in said formula (I),
R₁ represents a cyclopropyl radical, Y, R₂, R₄, R₅, R₆, R₇ and R₈ have the meaning already indicated, their tautomeric forms as well as their addition salts with mineral or organic bases.

3. N-(phenyl) 2-cyano-3-hyrdoxy-propenamides, corresponding to formula (I) according to claim 1 or 2, characterized in that in said formula (I), R2 represents a hydrogen atom or a methyl radical and R₁, Y, R₄, R₅, R₆, R₇ and R₈ have the meaning already indicated, their tautomer forms and their addition salts with mineral or organic bases.

4. N-(phenyl) 2-cyano-3-hydroxy-propenamides corresponding to formula (I) according to any one of claims 1, 2 and 3, characterized in that in said formula (I),
R₁ represents a cyclopropyl radical,
R₂ represents a hydrogen atom or a methyl radical,
Y represents a -(CH₂)ₘ- group in which m is equal to 0, 1 or 2 or a group: 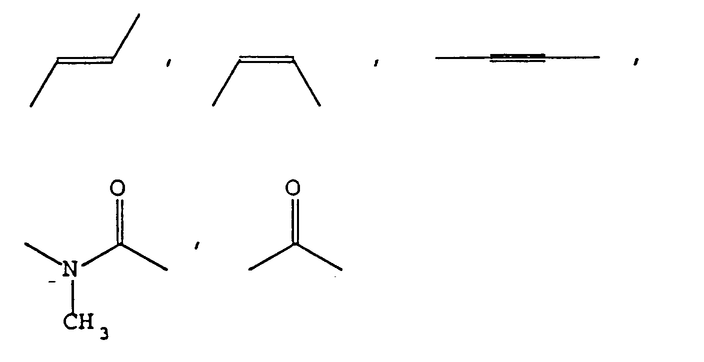 and either R₄, R₅, R₆, R₇ and R₈, identical or different, represent a hydrogen atom, a chlorine, bromine or fluorine atom, a -CN group, an -NO₂ group, a methyl radical, a cyclopropyl radical, a methoxy radical, a methylthio radical, a -(CH₂)ₙ-CF₃ radical, an -O-(CH₂)ₙ-CF₃ radical, an -S-(CH₂)ₙ-CF₃ radical, in which radicals n represents an integer comprised between 0 and 3 or a - CO - R₁₀ group in which R₁₀ represents a hydrogen atom, a methyl radical, a cyclopropyl radical, or R₆ and R₇, together form a 6,7-O-CH₂-O- group and R₄, R₅ and R₈ have the meaning indicated in claim 1, their tautomeric forms as well as their addition salts with mineral or organic bases.

5. N-(phenyl) 2-cyano-3-hydroxy-propenamides corresponding to formula (I) according to any one of claims 1, 2, 3 and 4, characterized in that in said formula (I),
R₁ represents a cyclopropyl radical, R₂ represents a hydrogen atom or a methyl radical,
Y represents a -(CH₂)ₘ- group in which m is equal to 0, 1 or 2 or a group: 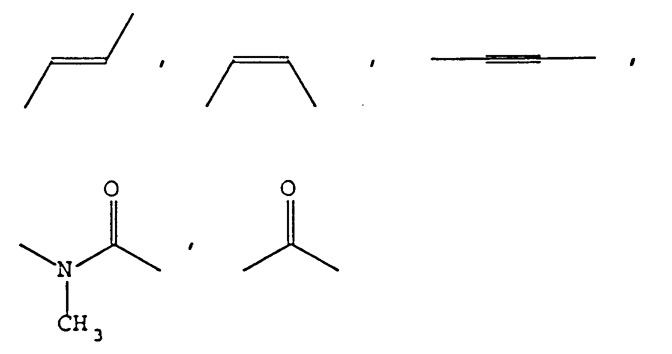
R₄, R₅, R₆, R₇ and R₈ , identical or different, represent a hydrogen atom, a chlorine, bromine or fluorine atom, an - NO₂ group, a methyl radical or a -CF₃ radical, their tautomeric forms, as well as their addition salts with the mineral or organic bases.

6. N-phenyl 2-cyano-3-hydroxy-propenamides corresponding to formula (I) according to any one of claims 1 to 5 the names of which follow:
- N-[4-(4'-chlorophenyl-E-ethenyl) phenyl] 2-cyano 3-cyclopropyl 3-hydroxy-prop-2-enamide,
- N-[4-(4'-fluorophenyl-E-ethenyl) phenyl] 2-cyano 3-cyclopropyl 3-hydroxy-prop-2-enamide,
- N-[4-biphenylyl] 2-cyano 3-cyclopropyl 3-hydroxy-prop-2-enamide,
- N-[4-(4'-fluorophenyl) phenyl] 2-cyano 3-cyclopropyl 3-hydroxy-prop-2-enamide,
as well as their addition salts with mineral or organic bases.

7. Process for the preparation of N-phenyl 2-cyano-3-hydroxy-propenamides as defined in formula (I) of claim 1, as well as their salts, characterized in that a product of formula (II): 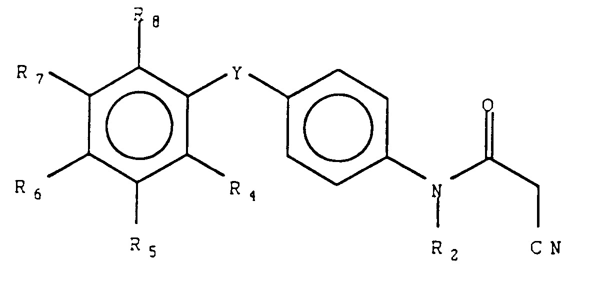 in which R₂, Y, R₄, R₅, R₆, R₇ and R₈, have the meaning already indicated in claim 1, is reacted successively with sodium hydride, if necessary in the presence of a catalyst, then the product is reacted with the functional derivative of an acid of formula (III): 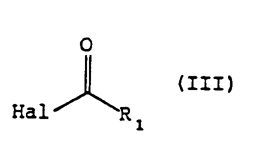 in which Hal represents a halogen atom and R₁ has the meaning already indicated in claim 1, in order to obtain a corresponding product of formula (I) which is isolated and if desired salified or reacted with a functional derivative of an acid of formula (III_{A}): 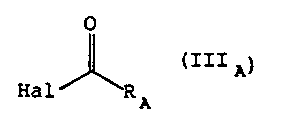 in which Hal represents a halogen atom and R_{A} represents a duly protected R₁ radical, in order to obtain a product of formula (I_{A}): 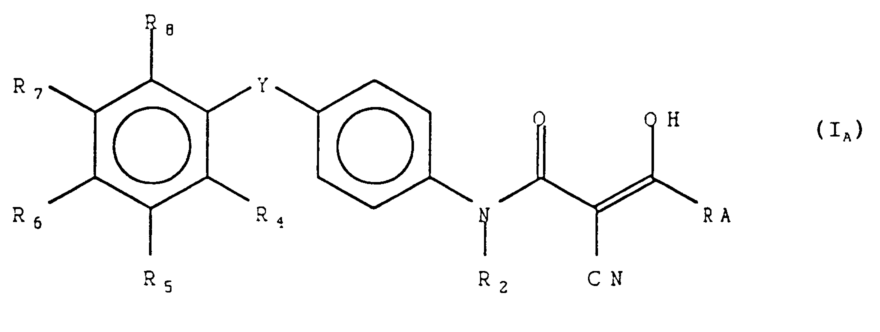 in which R_{A}, R₂, Y, R₄, R₅, R₆, R₇ and R₈ have the meaning already indicated, the product of formula (I_{A}) thus obtained in which R_{A} has the meaning already indicated is deprotected in order to obtain a corresponding product of formula (I), which is isolated and if desired salified.

8. Preparation process according to claim 7, characterized in that the reaction of the product of formula (II) with sodium hydride is carried out in the presence of imidazole, in an anhydrous organic solvent such as tetrahydrofuranne or dichloromethane.

9. Preparation process according to claim 7 or 8, characterized in that the reaction with the functional derivative of the acif of formula (III) or (III_{A}) is carried out at low temperature or at ambient temperature, in the presence of imidazole within an anhydrous organic solvent such as tetrahydrofuran or dichloromethane.

10. Preparation process according to any one of claims 7 to 9, characterized in that in the compound of formula (III_{A}), R_{A} represents an R₁ radical protected by an arylseleno group, preferably a phenylseleno radical.

11. Preparation process according to any one of claims 7 to 10, characterized in that the product of formula (II) is prepared by reacting a product of formula (IV): 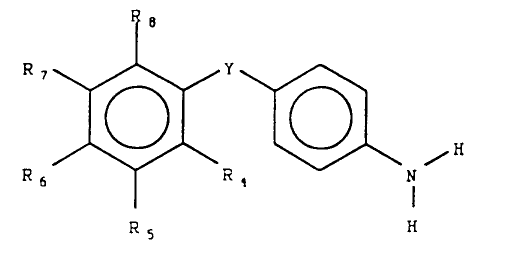 in which Y, R₄, R₅, R₆, R₇ and R₈ have the meaning indicated in claim 7 with cyanoacetic acid.

12. Preparation process according to claim 11, characterized in that the reaction of the compound of formula (IV) with cyanoacetic acid is carried out in the presence of dicyclocarbodiimide or phosphorus pentachloride in an anhydrous organic solvent such as tetrahydrofuran or dichloromethane.

13. Preparation process according to claim 11 or 12, characterized in that the compound of formula (IV) is prepared by the reduction of the corresponding nitro product of formula (V): 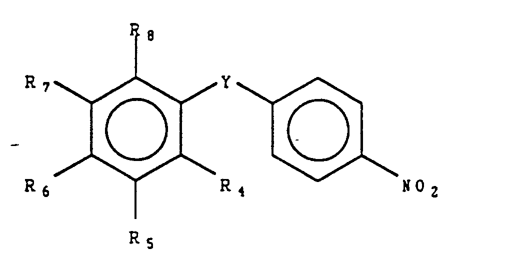 in which Y, R₄, R₅, R₆, R₇ and R₈ have the meaning indicated in claim 11.

14. Medicaments characterized in that they are constituted by N-phenyl 2-cyano-3-hydroxy-propenamides as defined in formula (I) of claim 1, as well as by their addition salts with pharmaceutically acceptable bases.

15. Medicaments, characterized in that they are constituted by N-phenyl 2-cyano-3-hydroxy-propenamides as defined in any one of claims 2 to 6, as well as their addition salts with pharmaceutically acceptable bases.

16. Pharmaceutical compositions, characterized in that they contain as active ingredient at least one of the medicaments as defined in any one of claims 14 and 15.

## Patentansprüche

1. N-(Phenyl)-2-cyano-3-hydroxy-propenamide der allgemeinen Formel (I) 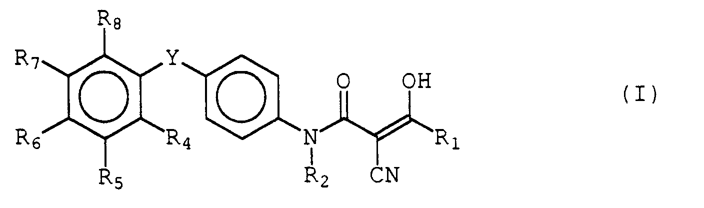 in der
- R₁ einen Rest Cycloalkyl mit 3 bis 6 Kohlenstoffatomen darstellt,
- R₂ ein Wasserstoffatom, einen Rest Alkyl mit 1 bis 3 Kohlenstoffatomen bedeutet,
- Y eine der folgenden Gruppen darstellt:
-NR₃-, -SO₂-NR₃-, -NR₃-SO₂-, -CHOH-, -CF₂-, -(CH₂)ₘ-, 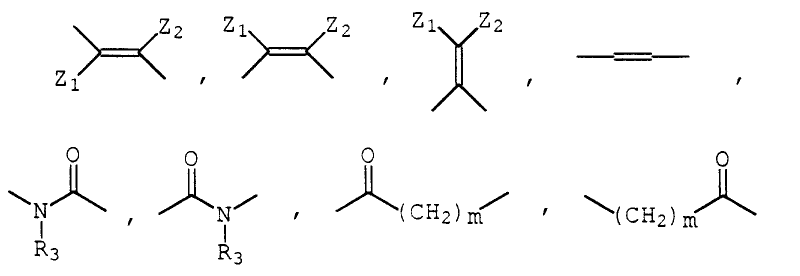 worin Z₁ und Z₂, gleich oder verschieden, ein Wasserstoffatom, ein Halogenatom, einen Rest Alkyl mit 1 bis 3 Kohlenstoffatomen, eine Gruppe CN, eine Gruppe NO₂ darstellen, R₃ ein Wasserstoffatom oder einen Rest Alkyl mit 1 bis 3 Kohlenstoffatomen bedeutet, m eine ganze Zahl ist, die zwischen 0 und 6 variieren kann,
- R₄, R₅, R₆, R₇ und R₈, gleich oder verschieden, ein Wasserstoffatom, ein Halogenatom, eine Gruppe -CN, eine Gruppe -NO₂, einen linearen oder verzweigten Rest Alkyl mit 1 bis 6 Kohlenstoffatomen, einen Rest Cycloalkyl mit 3 bis 6 Kohlenstoffatomen, einen linearen oder verzweigten Rest Alkoxy mit 1 bis 6 Kohlenstoffatomen, einen Rest Alkylthio mit 1 bis 6 Kohlenstoffatomen, einen Rest -(CH₂)ₙ-CX₃, einen Rest -O-(CH₂)ₙ-CX₃, einen Rest -S-(CH₂)ₙ-CX₃ darstellen, wobei in diesen Resten n eine ganze Zahl zwischen 0 und 3 ist und X ein Halogenatom oder eine Gruppe -CO-R₁₀, worin R₁₀ ein Wasserstoffatom ist, einen linearen oder verzweigten Rest Alkyl mit 1 bis 6 Kohlenstoffatomen oder einen Rest Cycloalkyl mit 3 bis 6 Kohlenstoffatomen bedeutet,
oder R₆ und R₇ zusammen eine Gruppe -O-CH₂-O- bilden und R₄, R₅ und R₈ die gleiche Bedeutung wie oben besitzen, ihre tautomeren Formen sowie ihre Additionssalze mit Mineralbasen oder organischen Basen.

2. N-(Phenyl)-2-cyano-3-hydroxy-propenamide der allgemeinen Formel (I) wie in Anspruch 1 definiert, dadurch gekennzeichnet, daß in der genannten Formel (I)
R₁ einen Rest Cyclopropyl darstellt und
Y, R₂, R₄, R₅, R₆, R₇ und R₈ die bereits angegebene Bedeutung besitzen, ihre tautomeren Formen sowie ihre Additionssalze mit Mineralbasen oder organischen Basen.

3. N-(Phenyl)-2-cyano-3-hydroxy-propenamide der allgemeinen Formel (I) wie in Anspruch 1 oder 2 definiert, dadurch gekennzeichnet, daß in der genannten Formel (I)
R₂ ein Wasserstoffatom oder einen Rest Methyl darstellt und R₁, Y, R₄, R₅, R₆, R₇ und R₈ die bereits angegebene Bedeutung besitzen, ihre tautomeren Formen sowie ihre Additionssalze mit Mineralbasen oder organischen Basen.

4. N-(Phenyl)-2-cyano-3-hydroxy-propenamide der allgemeinen Formel (I) wie in irgendeinem der Ansprüche 1, 2 oder 3 definiert, dadurch gekennzeichnet, daß in der genannten Formel (I)
R₁ einen Rest Cyclopropyl darstellt,
R₂ ein Wasserstoffatom oder einen Rest Methyl bedeutet,
Y eine Gruppe -(CH₂)ₘ- darstellt, worin m gleich 0, 1 oder 2 ist oder eine Gruppe 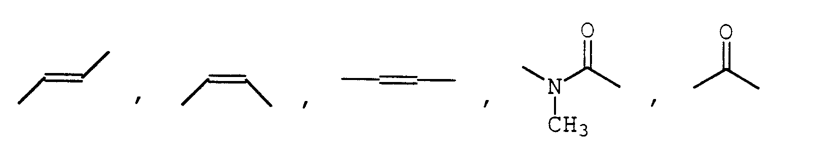 bedeutet, und entweder
R₄, R₅, R₆, R₇ und R₈, gleich oder verschieden, ein Wasserstoffatom, ein Atom von Chlor, Brom oder Fluor, eine Gruppe -CN, eine Gruppe -NO₂, einen Rest Methyl, einen Rest Cyclopropyl, einen Rest Methoxy, einen Rest Methylthio, einen Rest -(CH₂)ₙ-CF₃, einen Rest -O-(CH₂)ₙ-CF₃, einen Rest -S-(CH₂)ₙ-CF₃ darstellen, wobei in diesen Resten n eine ganze Zahl zwischen 0 und 3 ist, oder eine Gruppe -CO-R₁₀, worin R₁₀ ein Wasserstoffatom ist, einen Rest Methyl, einen Rest Cyclopropyl bedeuten, oder R₆ und R₇ zusammen eine Gruppe 6,7-O-CH₂-O- darstellen und R₄, R₅ und R₈ die bei Anspruch 1 angegebene Bedeutung besitzen, ihre tautomeren Formen sowie ihre Additionssalze mit Mineralbasen oder organischen Basen.

5. N-(Phenyl)-2-cyano-3-hydroxy-propenamide der allgemeinen Formel (I) nach irgendeinem der Ansprüche 1, 2, 3 oder 4, dadurch gekennzeichnet, daß in der genannten Formel (I)
R₁ einen Rest Cyclopropyl darstellt,
R₂ ein Wasserstoffatom oder einen Rest Methyl bedeutet,
Y eine Gruppe -(CH₂)ₘ- darstellt, worin m gleich 0, 1 oder 2 ist oder eine Gruppe 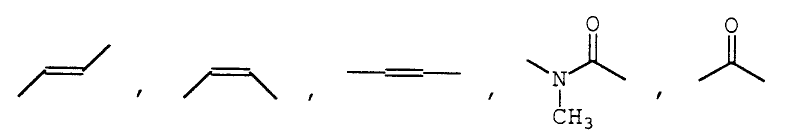 bedeutet,
R₄, R₅, R₆, R₇ und R₈, gleich oder verschieden, ein Wasserstoffatom, ein Atom von Chlor, Brom oder Fluor, eine Gruppe -NO₂, einen Rest Methyl oder einen Rest -CF₃ darstellen, ihre tautomeren Formen sowie ihre Additionssalze mit Mineralbasen oder organischen Basen.

6. N-(Phenyl)-2-cyano-3-hydroxy-propenamide der allgemeinen Formel (I) nach irgendeinem der Ansprüche 1 bis 5 mit den folgenden Namen:
- N-[4-(4'-Chlorphenyl-E-ethenyl)-phenyl]-2-cyano-3-cyclopropyl-3-hydroxy-prop-2-enamid,
- N-[4-(4'-Fluorphenyl-E-ethenyl)-phenyl]-2-cyano-3-cyclopropyl-3-hydroxy-prop-2-enamid,
- N-[4-(Biphenyl)]-2-cyano-3-cyclopropyl-3-hydroxy-prop-2-enamid,
- N-[4-(4'-Fluorphenyl)-phenyl]-2-cyano-3-cyclopropyl-3-hydroxy-prop-2-enamid,
sowie ihre Additionssalze mit Mineralbasen oder organischen Basen.

7. Verfahren zur Herstellung von N-(Phenyl)-2-cyano-3-hydroxy-propenamiden der allgemeinen Formel (I) nach Anspruch 1 sowie ihren Salzen, dadurch gekennzeichnet, daß man ein Produkt der Formel (II) 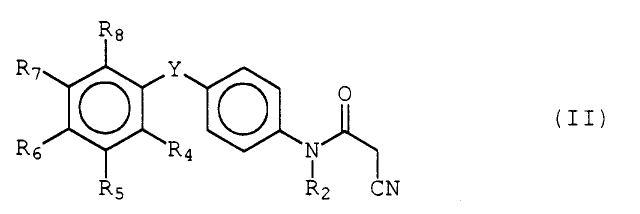 in der R₂, Y, R₄, R₅, R₆, R₇ und R₈ die bereits in Anspruch 1 angegebene Bedeutung besitzen, nacheinander mit Natriumhydrid, wenn erforderlich in Anwesenheit eines Katalysators, und anschließend das Produkt mit dem funktionellen Derivat einer Säure der Formel (III) 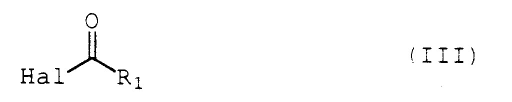 zur Reaktion bringt, in der Hal ein Halogenatom ist und R₁ die bereits in Anspruch 1 angegebene Bedeutung besitzt, um das entsprechende Produkt der Formel (I) zu erhalten, das man isoliert und wenn gewünscht, in Salz überführt,
oder mit dem funktionellen Derivat einer Saure der Formel (III_{A}) 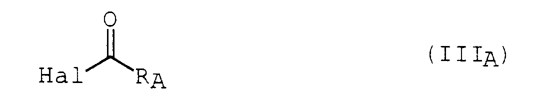 zur Reaktion bringt, in der Hal ein Halogenatom ist und R_{A} einen ordnungsgemäß geschützten Rest R₁ darstellt, um ein Produkt der Formel (I_{A}) 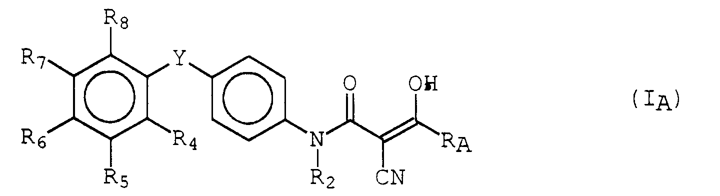 zu erhalten, in der R_{A}, R₂, Y, R₄, R₅, R₆, R₇ und R₈ die bereits angegebene Bedeutung besitzen, und man von dem auf diese Weise erhaltenen Produkt der Formel (I_{A}), in der R_{A} die bereits angegebene Bedeutung besitzt, die Schutzgruppen entfernt, um das entsprechende Produkt der Formel (I) zu erhalten, das man isoliert und wenn erwünscht, in Salz überführt.

8. Verfahren zur Herstellung nach Anspruch 7, dadurch gekennzeichnet, daß die Reaktion des Produktes der Formel (II) mit dem Natriumhydrid in Anwesenheit von Imidazol und in einem wasserfreien organischen Lösungsmittel wie Tetrahydrofuran oder Dichlormethan durchgeführt wird.

9. Verfahren zur Herstellung nach Anspruch 7 oder 8, dadurch gekennzeichnet, daß die Reaktion mit dem funktionellen Derivat der Säure der Formel (III) oder (III_{A}) bei niedriger Temperatur oder bei Umgebungstemperatur und in Anwesenheit von Imidazol sowie in einem wasserfreien organischen Lösungsmittel wie Tetrahydrofuran oder Dichlormethan durchgeführt wird.

10. Verfahren zur Herstellung nach irgendeinem der Ansprüche 7 bis 9, dadurch gekennzeichnet, daß in der Verbindung der Formel (III_{A}) R_{A} einen Rest R₁ darstellt, der durch eine Gruppe Arylseleno, vorzugsweise einen Rest Phenylseleno geschützt ist.

11. Verfahren zur Herstellung nach irgendeinem der Ansprüche 7 bis 10, dadurch gekennzeichnet, daß das Produkt der Formel (II) durch Reaktion eines Produktes der Formel (IV) 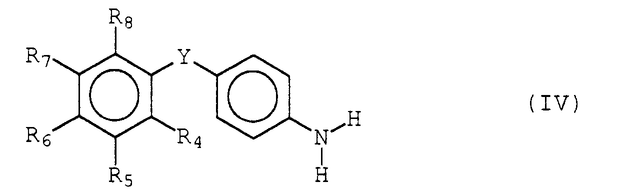 in der Y, R₄, R₅, R₆, R₇ und R₈ die bereits in Anspruch 7 angegebene Bedeutung besitzen, mit Cyanoessigsäure hergestellt wird.

12. Verfahren zur Herstellung nach Anspruch 11, dadurch gekennzeichnet, daß die Reaktion der Verbindung der Formel (IV) mit der Cyanoessigsäure in Anwesenheit von Dicyclocarbodiimid oder Phosphorpentachlorid in einem wasserfreien organischen Lösungsmittel wie Tetrahydrofuran oder Dichlormethan durchgeführt wird.

13. Verfahren zur Herstellung nach Anspruch 11 oder 12, dadurch gekennzeichnet, daß die Verbindung der Formel (IV) durch Reduktion des entsprechenden, nitrierten Produktes der Formel (V) 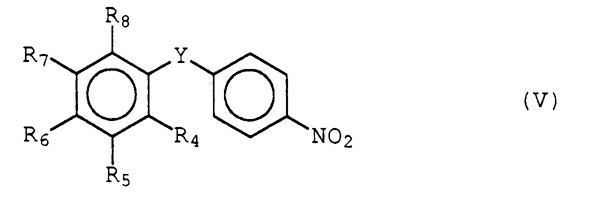 hergestellt wird, in der Y, R₄, R₅, R₆, R₇ und R₈ die bereits in Anspruch 11 angegebene Bedeutung besitzen.

14. Arzneimittel, dadurch gekennzeichnet, daß sie aus N-(Phenyl)-2-cyano-3-hydroxy-propenamiden wie in Formel (I) von Anspruch 1 definiert sowie ihren Additionssalzen mit pharmazeutisch akzeptablen Basen bestehen.

15. Arzneimittel, dadurch gekennzeichnet, daß sie aus N-(Phenyl)-2-cyano-3-hydroxy-propenamiden wie in irgendeinem der Ansprüche 2 bis 6 definiert sowie ihren Additionssalzen mit pharmazeutisch akzeptablen Basen bestehen.

16. Pharmazeutische Zusammensetzungen, dadurch gekennzeichnet, daß sie als Wirkstoff mindestens ein Arzneimittel wie in irgendeinem der Ansprüche 14 und 15 definiert umfassen.
